# EUROPÄISCHE PATENTANMELDUNG

(11) **EP 0 286 144 A2**
(43) Veröffentlichungstag der Anmeldung: **12.10.1988**
(21) Anmeldenummer: 88105732.7
(22) Anmeldetag: 11.04.1988
(51) Int. Cl.: C07D 501/36, A61K 31/545

(54) **Heterocyclylthiomethyl-cephalosporine**

(30) Priorität: 10.04.1987 CH 1407/87; 30.04.1987 CH 1657/87; 26.06.1987 CH 2414/87
(71) Anmelder: F. HOFFMANN-LA ROCHE AG, 4002 Basel (CH)
(72) Erfinder: Furlenmeier, André, Dr., CH-4058 Basel (CH); Götschi, Erwin, Dr., CH-4153 Reinach (CH); Hebeisen, Paul, Dr., CH-4153 Reinach (CH); Hofheinz, Werner, Dr., CH-4103 Bottmingen (CH); Link, Helmut, Dr., CH-4056 Basel (CH)
(74) Vertreter: Lederer, Franz, Dr.

(57) **Zusammenfassung**

Acylderivate der allgemeinen Formel 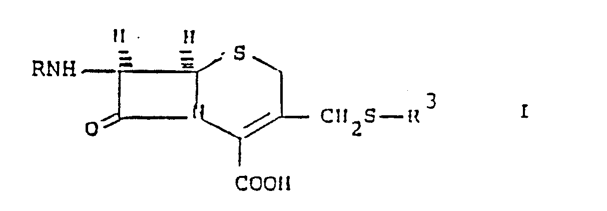 in der R einen Acylrest darstellt,
ausgenommen den Acylrest der allgemeinen Formel 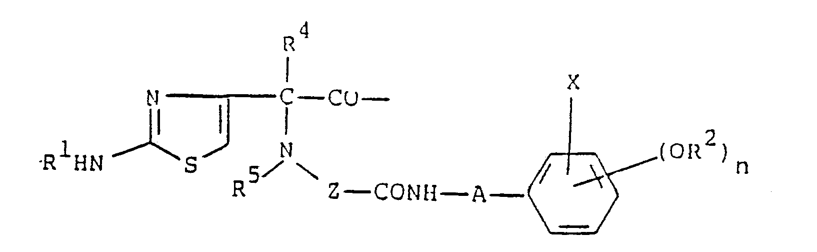 in der A eine Gruppe der allgemeinen Formeln
-NHCO-, -NHCONHCO-, -NHCOCH=CH-,-N= 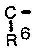

bedeutet, worin R⁶ Wasserstoff oder niederes Alkyl darstellt; R¹ und R² jeweils Wasserstoff oder eine Schutzgruppe, X Wasserstoff, Halogen, niederes Alkoxy, Nitro oder eine zusätzliche Gruppe -OR², n die Zahl 1 oder 2, R⁴ und R⁵ beide Wasserstoff oder zusammen eine zusätzliche Bindung und Z eine direkte Bindung oder Carbonyl (falls R⁴ und R⁵ beide Wasserstoff darstellen) oder eine Gruppe der Formel -O-B- (falls R⁴ und R⁵ zusammen eine zusätzliche Bindung darstellen) bedeutet, worin B geradkettiges, verzweigtes oder cyclisches niederes Alkylen bedeutet;
und worin ferner R³ eine substituierte bicyclische Gruppe der allgemeinen Formeln 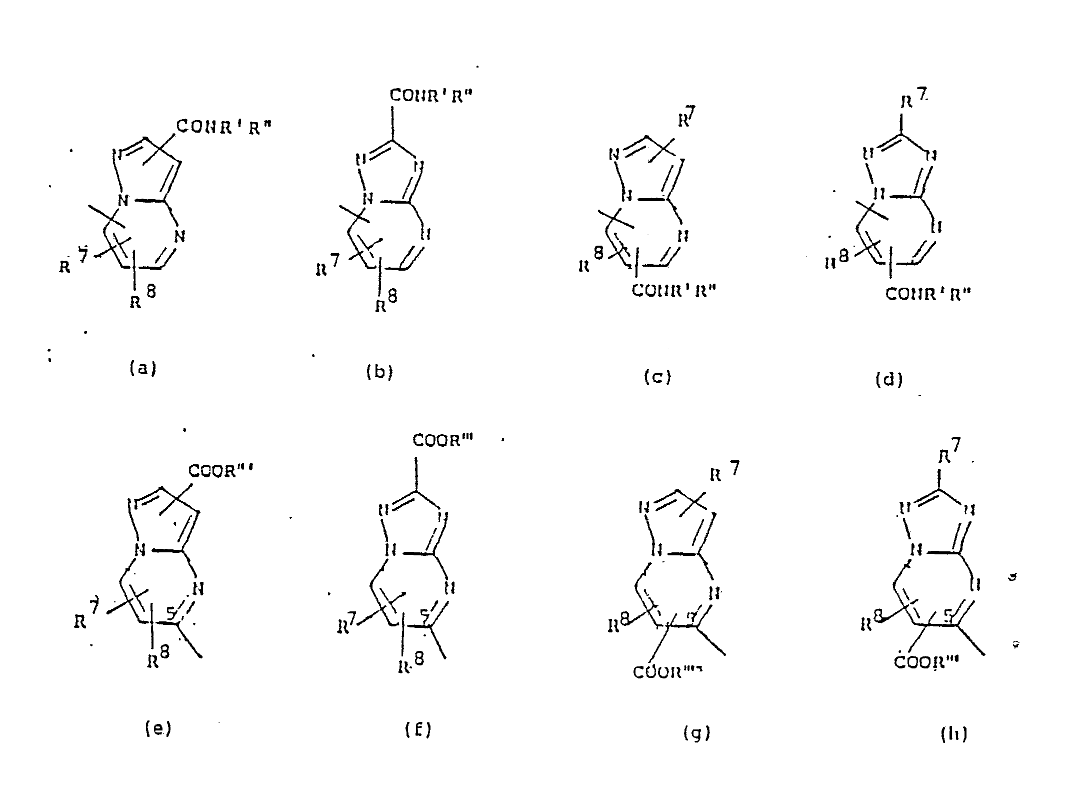 bedeutet, worin R⁷ und R⁸ je einzeln Wasserstoff, niederes Alkyl oder Trifluormethyl oder (in den Formeln (a), (b), (e) und (f)) zusammen Alkylen mit 3 oder 4 Kohlenstoffatomen darstellen, und Rʹ, Rʺ R‴ je einzeln Wasserstoff, niederes Alkyl oder niederes Cycloalkyl bedeuten; wobei Verbindungen der Formel I, worin R⁴ und R⁵ zusammen eine zusätzliche Bindung darstellen, in der syn-isomeren Form vorliegen bzw. als Gemisch mit der anti-isomeren Form, worin die syn-isomere Form überwiegt, sowie leicht hydrolysierbare Ester und Salze dieser Verbindungen und Hydrate der Verbindungen der Formel I bzw. von deren Estern und Salzen.

Die Verbindungen sind antibiotisch wirksam.

## Beschreibung

Die vorliegende Erfindung betrifft neue Acylderivate, und zwar Cephalosporinderivate der allgemeinen Formel 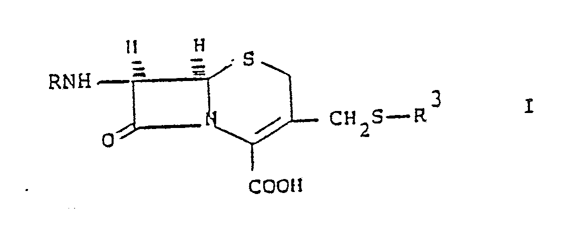 in der R einen Acylrest darstellt,
ausgenommen den Acylrest der allgemeinen Formel 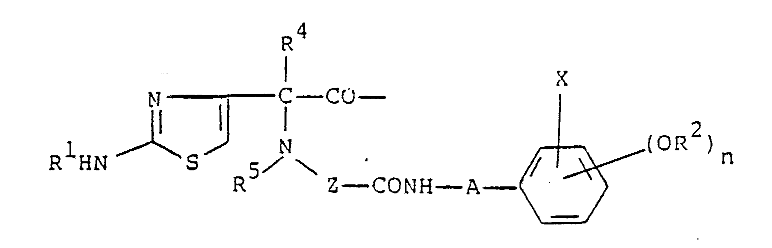 in der A eine Gruppe der allgemeinen Formeln
-NHCO-, -NHCONHCO-, -NHCOCH=CH-, -N= 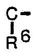

bedeutet, worin R⁶ Wasserstoff oder niederes Alkyl darstellt; R¹ und R² jeweils Wasserstoff oder eine Schutzgruppe, X Wasserstoff, Halogen, niederes Alkoxy, Nitro oder eine zusätzliche Gruppe -OR², n die Zahl 1 oder 2, R⁴ und R⁵ beide Wasserstoff oder zusammen eine zusätzliche Bindung und Z eine direkte Bindung oder Carbonyl (falls R⁴ und R⁵ beide Wasserstoff darstellen) oder eine Gruppe der Formel -O-B- (falls R⁴ und R⁵ zusammen eine zusätzliche Bindung darstellen) bedeutet, worin B geradkettiges, verzweigtes oder cyclisches niederes Alkylen bedeutet;
und worin ferner R³ eine substituierte bicyclische Gruppe der allgemeinen Formeln 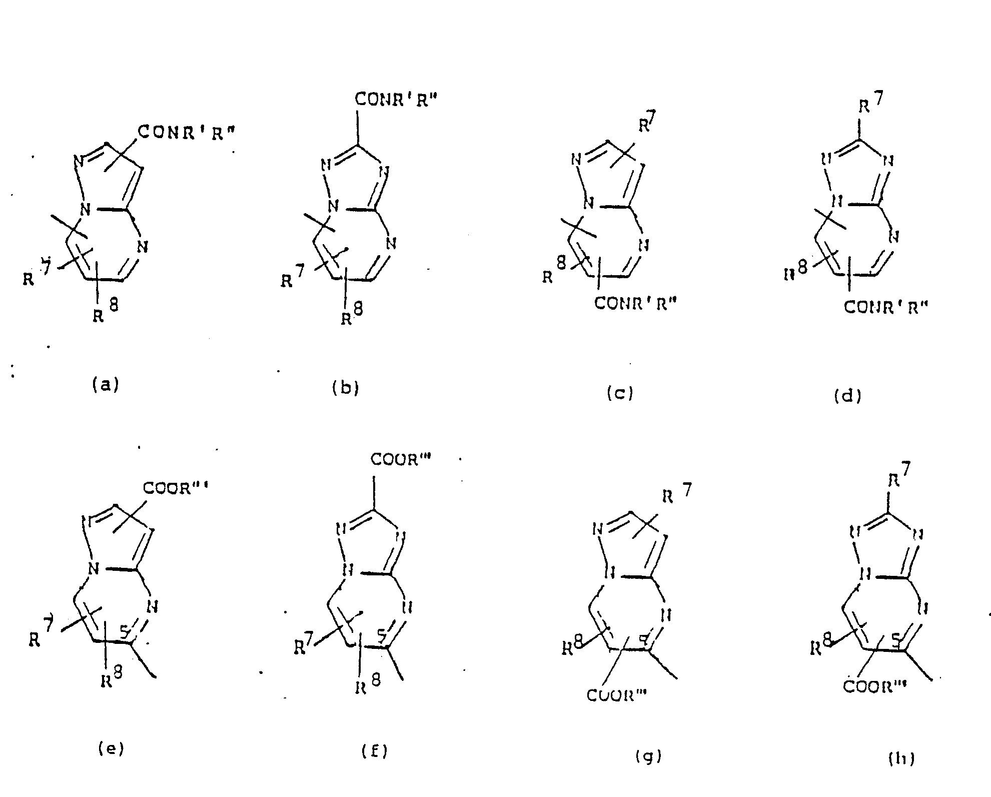 bedeutet, worin R⁷ und R⁸ je einzeln Wasserstoff, niederes Alkyl oder Trifluormethyl oder (in den Formeln (a), (b), (e) und (f)) zusammen Alkylen mit 3 oder 4 Kohlenstoffatomen darstellen, und Rʹ, Rʺ und R‴ je einzeln Wasserstoff, niederes Alkyl oder niederes Cycloalkyl bedeuten,
sowie leicht hydrolysierbare Ester und Salze dieser Verbindungen und Hydrate der Verbindungen der Formel I bzw. von deren Estern und Salzen.

Ausgeschlossen von der vorliegenden Erfindung sind die Verbindungen der allgemeinen Formel 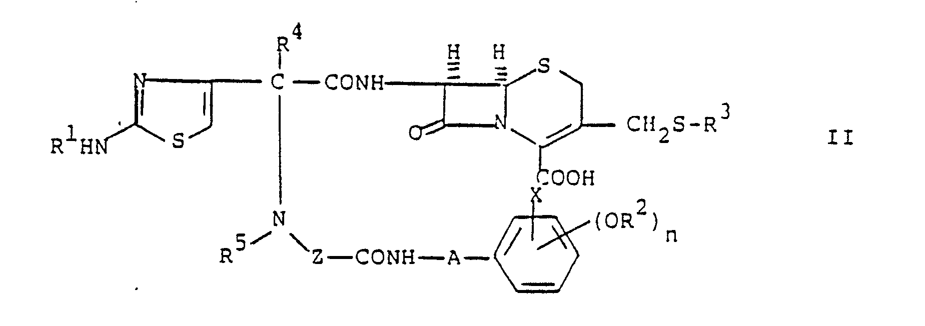 worin R¹-R⁵, A, Z und n die obige Bedeutung haben
sowie leicht hydrolysierbare Ester und Salze dieser Verbindungen und Hydrate der Verbindungen der Formel II bzw. von deren Estern und Salzen.

Gegenstand der vorliegenden Erfindung sind somit diejenigen Acylderivate der Formel I, worin R einen anderen Acylrest als denjenigen von Formel II darstellt, sowie leicht hydrolysierbare Ester und Salze diesen Verbindungen und Hydrate solcher Verbindungen der Formel I bzw. von deren Estern und Salzen.

Eine Untergruppe dieser erfindungsgemässen Verbindungen sind Verbindungen der allgemeinen Formel 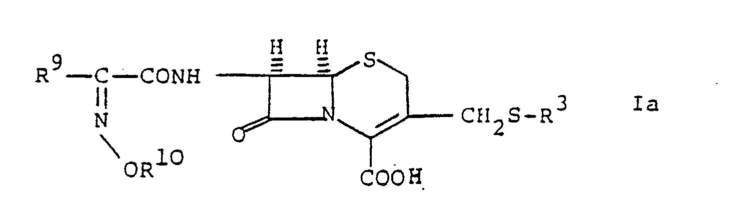 in der R³ die obige Bedeutung hat, R⁹ einen gegebenenfalls substituierten 5- oder 6-gliedrigen Heterocyclus mit 1-4, vorzugsweise 1 oder 2 Heteroatomen, wie N, O, und/oder S, darstellt und R¹⁰ Wasserstoff, niederes Alkanoyl, niederes Alkyl , niederes Alkenyl, niederes Cycloalkyl, niederes Cycloalkenyl oder niederes Alkyl substituiert durch Carboxy, Carbamoyl, niederes Alkoxycarbonyl oder niederes Alkanoyl bedeutet, oder R¹⁰ einen Rest der allgemeinen Formeln
-P-Q -P-CO-Q -P-CO-NH-NH-CO-Q¹
bedeutet, worin P niederes Alkylen oder niederes Cycloalkylen bedeutet, Q eine der Gruppen 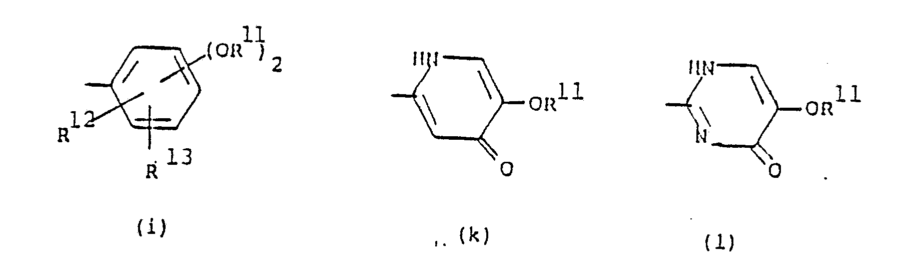 darstellt, worin R¹¹ Wasserstoff, niederes Alkanoyl oder tri(niederes Alkyl)-Silyl, R¹² Wasserstoff, -OR¹¹, niederes Alkoxy, Halogen, -OCOR¹⁴,-OCOOR¹⁴, -N(R¹⁴)₂, -NH-COR¹⁴, -NHCOOR¹⁴ -COR¹⁴, -SR¹⁴, -SOR¹⁴, -SO₂R¹⁴, -SO₃H, -COOR¹⁴, -CON(R¹⁴)₂ oder Nitro, R¹³ Wasserstoff oder Halogen und R¹⁴ Wasserstoff oder niederes Alkyl bedeutet, wobei in der Gruppe (i) die beiden Reste -OR¹¹ in 2,3- oder 3,4-Stellung stehen:
und Q¹ eine der letztgenannten Gruppen (k) und (l) bedeutet,
sowie leicht hydrolysierbare Ester und Salze dieser Verbindungen und Hydrate der Verbindungen der Formel Ia bzw. von deren Estern und Salzen.

Die Verbindungen der Formel Ia liegen in der syn-isomeren Form vor bzw. als Gemisch mit der anti-isomeren Form, worin die syn-isomere Form überwiegt.

Beispiele für 5- bzw. 6-gliedrige Heterocyclen R⁹ sind Thienyl, Furyl, Oxazolyl, Pyrazolyl, Pyridinyl, Pyrazinyl, Thiazolyl, Pyrimidinyl, Thiadiazolyl. Beispiele für Substituenten auf diesen Heterocyclen sind: Halogen, Hydroxy, Nitro, Amino, Cyano, Trifluormethyl, niederes Alkyl und niederes Alkoxy.

Bevorzugte Heterocyclen R⁹ sind 2-Amino-4-thiazolyl und 5-Amino-3-thiadiazolyl.

"Niederes Alkyl" und "niederes Alkenyl" bedeuten geradkettige und verzweigte Kohlenstoffketten mit 1-7, insbesondere 1-4 Kohlenstoffatomen, z.B. Methyl, Aethyl, n-Propyl, Isopropyl, t-Butyl, Vinyl, Allyl, 2-Butenyl oder dergleichen. Abgewandelte Gruppen, wie niederes Alkoxy (niederes Alkyl-O), niederes Alkanoyl (niederes Alkyl-CO-), niederes Alkoxycarbonyl (niederes Alkyl-OCO-) und niederes Alkanoyloxy (niederes Alkyl-COO-), haben analoge Bedeutung, auch in Zusammensetzung, wie niederes Alkanoyloxyalkyl. Niederes Cycloalkyl und niederes Cycloalkenyl haben 3-7 Kohlenstoffatome, wie z.B. Cyclopropyl, Cyclobutyl, Cyclohexyl, Methyl-cyclopropyl, Cyclohexenyl. "Niederes Alkylen" ist analog zu "niederem Alkyl" zu verstehen und hat 1-7, insbesondere 1-4 Kohlenstoffatome, z.B.

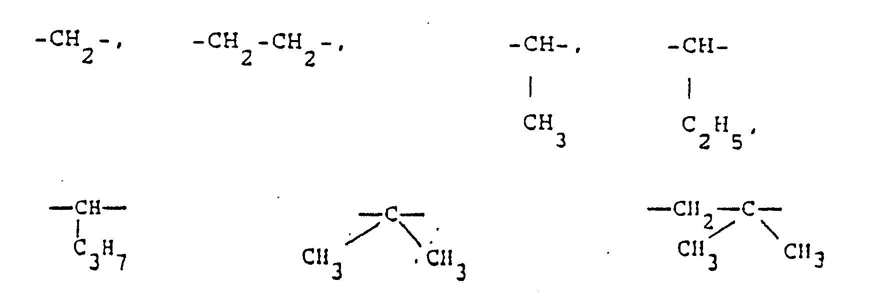

"Niederes Cycloalkylen" bzw. "cyclisches niederes Alkylen" ist analog zu "niederem Cycloalkyl" zu verstehen und hat 3-7 Kohlenstoffatome, z.B. 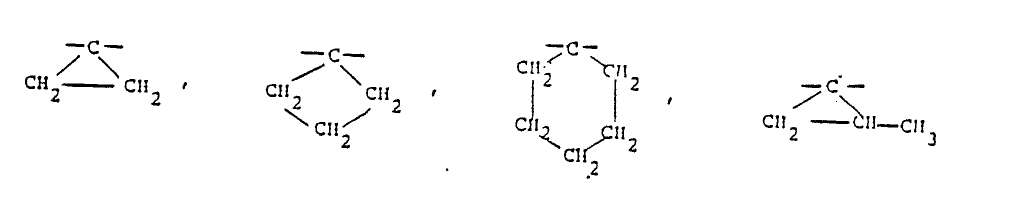 "Halogen" stellt Chlor, Fluor, Brom und Jod der, insbesondere Chlor.

Bevorzugte Gruppen R¹⁰sind:
Methyl, Allyl, Carboxymethyl, Carbamoylmethyl, 1-Carboxy-1-methyläthyl, Pivaloyl sowie die Gruppen
-P¹-Q -P¹-CO-Q -P¹-CO-NH-NH-CO-Q¹
worin Q die obige Bedeutung hat und P¹ die Gruppe 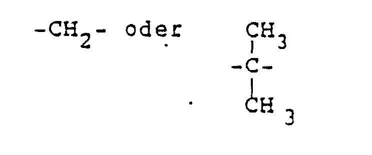 darstellt,
insbesondere die Gruppen 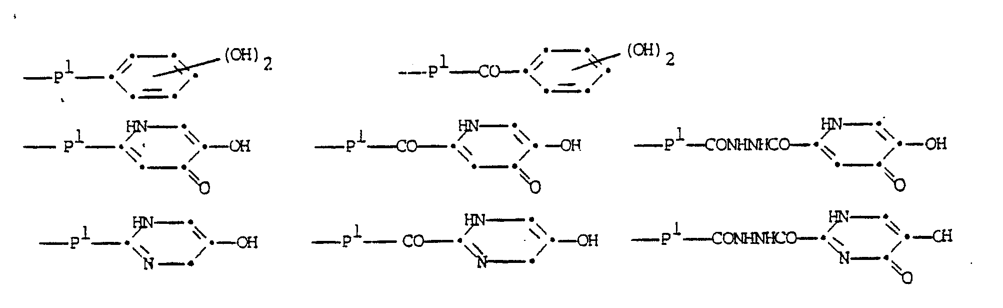 worin P¹ die obige Bedeutung hat und die beiden durch (OH)₂ bezeichneten Hydroxygruppen in 2,3- oder 3,4-Stellung stehen.

Die bicyclischen Reste (a)-(d) sind bevorzugt über die Stellung 5: 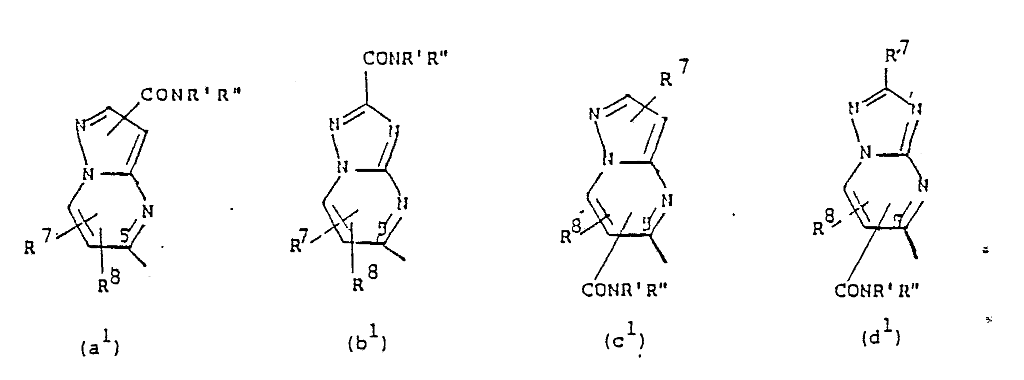 oder, insbesondere, über die Stellung 7: 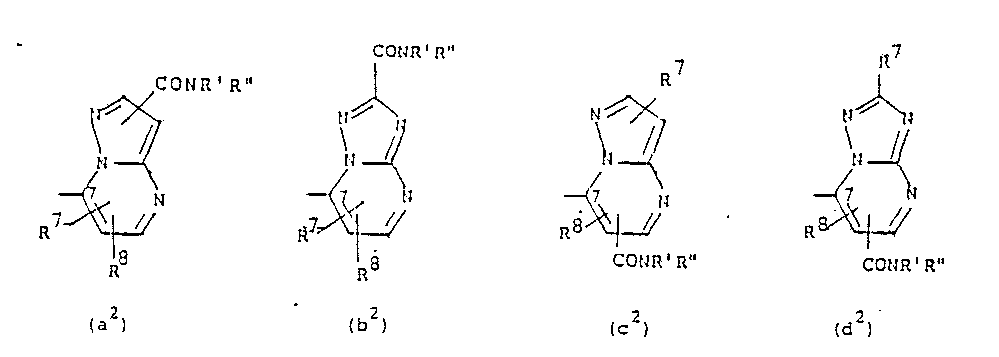 verknüpft.

Der Substituent -CONRʹRʺ der Gruppe (a) und der Substituent -COOR‴ der Gruppe (e) stehen vorzugsweise in Stellung 3:

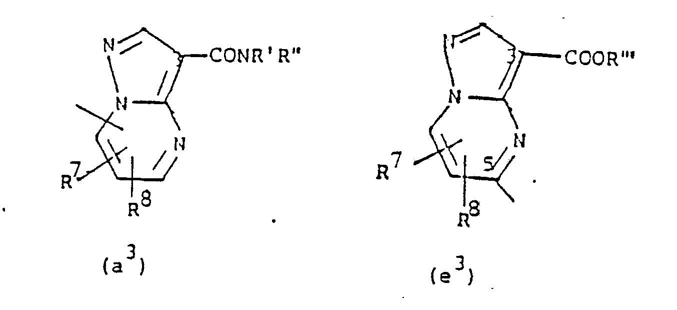

Eine bevorzugte Untergruppe der erfindungsgemässen Verbindungen sind diejenigen, worin R³ eine substituierte bicyclische Gruppe der allgemeinen Formeln 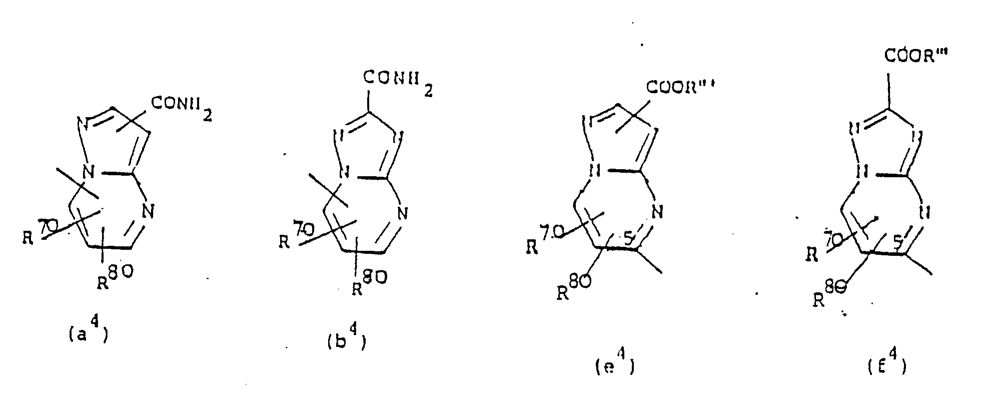 bedeutet, worin R⁷⁰ und R⁸⁰ je einzeln Wasserstoff, Methyl oder Trifluormethyl oder zusammen Alkylen mit 3 oder 4 Kohlenstoffatomen darstellen und R‴ Wasserstoff oder Methyl bedeutet.

Bevorzugte Gruppen R³ sind 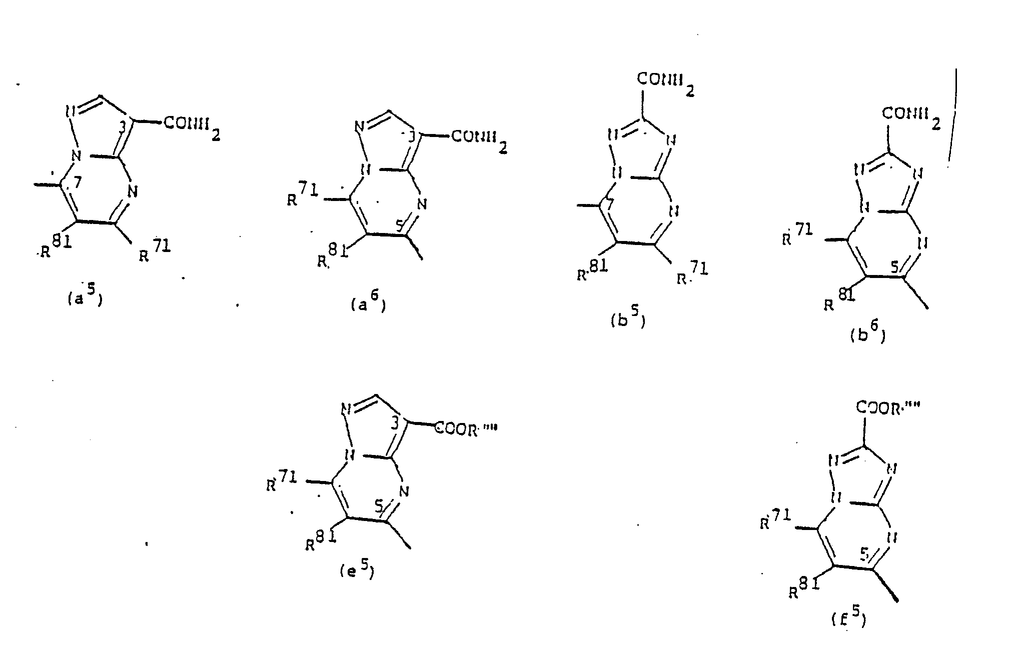 worin R⁷¹ Methyl oder Trifluormethyl und R⁸¹ Wasserstoff oder R⁷¹ und R⁸¹ zusammen Tetramethylen darstellen und Rʺʺ Wasserstoff oder Methyl bedeutet.

Besonders bevorzugte Gruppen R³ sind

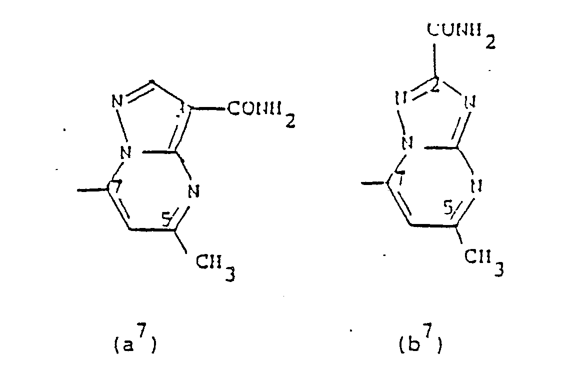

Salze der Verbindungen der Formel I sind pharmazeutisch verträgliche Salze und können Salze mit Basen oder mit Säuren sein. Salze mit Basen sind z.B. Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit Diisopropylamin, Benzylamin, Dibenzylamin, Triäthanolamin, Triäthylamin, N,N-Dibenzyläthylendiamin, N-Methylmorpholin, Pyridin, Piperazin, N-Aethyl-piperidin, Procain. Die Verbindungen der Formel I bilden ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dergleichen, Alkyl- und Mono-arylsulfonate, wie Aethansulfonate, Toluolsulfonate, Benzolsulfonate und dergleichen, und auch andere organische Säuresalze, wie Acetate, Tartrate, Maleate, Citrate, Benzoate, Salicylate, Ascorbate und dergleichen.

Als leicht hydrolysierbare Ester der Verbindungen der Formel I sind Verbindungen der Formel I zu verstehen, deren Carboxygruppe in Form einer leicht hydrolysierbaren Estergruppe vorliegt. Beispiele solcher Ester, die herkömmlicher Art sein können, sind die niederen Alkanoyloxyalkylester, z.B. der Acetoxymethyl-, Pivaloyloxymethyl-, 1-Acetoxyäthyl- und 1- Pivaloyloxyäthylester; die niederen Alkoxycarbonyloxyalkylester, z.B. der Methoxycarbonyloxymethyl-, 1-Aethoxycarbonyloxyäthyl- und 1-Isopropoxycarbonyloxyäthylester; die Lactonylester, z.B. der Phthalidyl- und Thiophthalidylester; die niederen Alkoxymethylester, z.B. der Methoxymethylester; und die niederen Alkanoylaminomethylester, z.B. der Acetamidomethylester. Auch andere Ester, z.B. die Benzyl- und Cyanmethylester, können brauchbar sein. Allenfalls vorhandene, zusätzliche Carboxygruppen in einer Verbindung der Formel I können ebenfalls die obigen leicht hydrolysierbaren Ester bilden.

Die Verbindungen der Formel I (einschliesslich deren Salze und leicht hydrolysierbare Ester) können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Bevorzugte Produkte sind:
(6R,7R)-7-[(Z)-2-(2-Amino-4-thiazolyl)-2-(methoxyimino]-acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[t-butoxycarbonyl)methoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[(pivaloyloxy)-imino]acetamido]-3-[[[2-carbamoyl-5-methyl-s-triazolo[1,5-a]-pyrimidin-7-yl]thio]methyl]-8-oxo-5-thia-1-azabicyclo[4.2.0]-oct-2-en-2-carbonsäure,
(6R,7R)-7-[(Z)-2-(2-Amino-4-thiazolyl)-2-[[1-(t-butoxycarbonyl)-1-methyläthoxy]imino]acetamido]-3-[[[2-carbamoyl-5-methyl-s-triazolo[1,5-a]pyrimidin-7-yl]thio]-methyl]-8-oxo-5-thia-1-azabicyclo[4.2.0]oct-2-en-2-carbonsäure,
(6R,7R)-7-[(Z)-2-(2-Amino-4-thiazolyl)-2-[(carboxymethoxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[1-(carboxy-1-methyläthoxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[(carbamoylmethoxy)imino]acetamido]-3-[[(2-carbamoyl-5-methyl-s-triazolo-[1,5-a]pyrimidin-7-yl)thio]methyl]-8-oxo-5-thia-1-azabicyclo-[4.2.0]oct-2-en-2-carbonsäure,
(6R,7R)-7-[(Z)-2-(2-Amino-4-thiazolyl)-2-[[1-[3-[(1,4-dihydro-5-hydroxy-4-oxo-2-pyridyl)carbonyl]carbazoyl]-1-methyläthoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[(1,4-dihydro-5-hydroxy-4-oxo-2-pyrimidinyl)methoxy]imino]acetamido]-3-[[(2-carbamoyl-5-methyl-s-triazolo[1,5-a]pyrimidin-7-yl)thio]-methyl]-8-oxo-5-thia-1-azabicyclo[4.2.0]oct-2-en-2-carbonsäure,
sowie Salze dieser Verbindungen.

Die obigen Acylderivate werden erfindungsgemäss dadurch hergestellt, dass man
(a) eine Verbindung der allgemeinen Formel 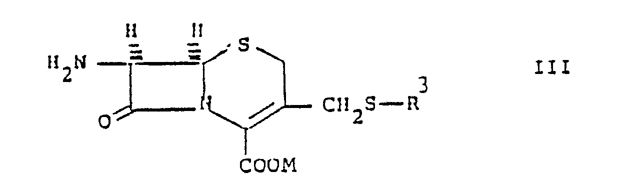 in der R³ die oben gegebene Bedeutung hat und M Wasserstoff oder eine Esterschutzgruppe darstellt,
   mit einem Acylierungsmittel umsetzt und allfällige Schutzgruppe gewünschtenfalls abspaltet, oder dass man
(b) eine Verbindung der allgemeinen Formel 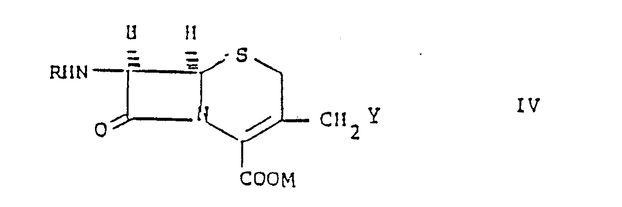 in der R und M die oben gegebene Bedeutung haben und Y eine Abgangsgruppe darstellt,
   mit einer Verbindung der allgemeinen Formel

   HSR³ V

   in der R³ die oben gegebene Bedeutung hat,
   umsetzt und allfällige Schutzgruppen gewünschtenfalls abspaltet, oder dass man
(c) zur Herstellung von Verbindungen der Formel I, worin R die allgemeine Formel 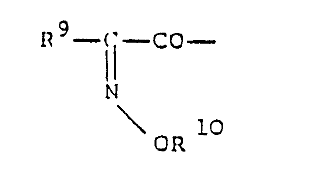 worin R⁹ und R¹⁰ die oben gegebene Bedeutung haben, hat, eine Verbindung der allgemeinen Formel 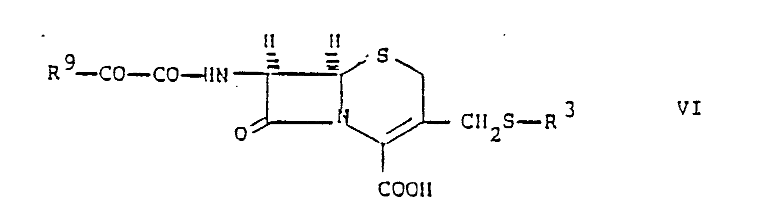 worin R³ und R⁹ die oben gegebene Bedeutung haben, mit einem Salz einer Verbindung der allgemeinen Formel

   NH₂OR¹⁰ VII

   worin R¹⁰ die oben gegebene Bedeutung hat,
   umsetzt, oder dass man
(d) zur Herstellung eines leicht hydrolysierbaren Esters einer Verbindung der Formel I eine Carbonsäure der Formel I einer entsprechenden Veresterung unterwirft, oder dass man
(e) zur Herstellung von Salzen und Hydraten eine Verbindung der Formel I bzw. Hydraten dieser Salze eine Verbindung der Formel I in ein Salz oder Hydrat bzw. ein Hydrat dieses Salzes überführt.

Allfällige reaktive Gruppen der oben eingesetzten Ausgangsverbindungen können gegebenenfalls geschützt sein. So können z.B. phenolische Hydroxygruppen durch Acetyl, Trimethylsilyl oder Tetrahydropyranyl und Aminogruppen durch z.B. sauer abspaltbare Schutzgruppen, z.B. t-Butoxycarbonyl oder Trityl, oder auch basisch-hydrolytisch abspaltbare Schutzgruppen, wie z.B. Trifluoracetyl, oder durch mit Thioharnstoff abspaltbare Schutzgruppen, wie z.B. Chlor-, Brom- und Jodacetyl, geschützt sein. Carboxygruppen können durch z.B. Benzhydryl-, t-Butyl-, Allyl- oder Trimethylsilylgruppen geschützt sein.

Gemäss Variante (a) des erfindungsgemässen Verfahrens wird eine Verbindung der allgemeinen Formel III acyliert. Als Acylierungsmittel kommen in Frage: entsprechende Carbonsäuren in Gegenwart von 2-Halogenpyridiniumsalzen, z.B. von 2-Chlor- oder 2-Fluor-2-methylpyridiniumchlorid oder -tosylat, oder auch in Gegenwart von N,N-Dicyclohexylcarbodiimid, bevorzugt zusammen mit N-Hydroxybenztriazol oder N-Hydroxysuccinimid. Es können auch entsprechende reaktionsfähige Derivate der Carbonsäure eingesetzt werden, wie z.B. das Säurehalogenid, Säureanhydrid oder Säureazid. Ebenfalls verwendbar sind die entsprechenden Thiolester, wie z.B. 2-Benzthiazolylthioester, wie auch Hydroxybenztriazolester oder N-Hydroxysuccinimidester. Allfällige Esterschutzgruppen in Verbindungen III sind z.B. Benzhydryl-, t-Butyl-, Allyl-oder Trimethylsilylgruppen. Die Umsetzung wird vorzugsweise in einem organischen Lösungsmittel oder Lösungsmittelgemisch, gegebenenfalls im Gemisch mit Wasser, durchgeführt, z.B. Aceton, Methylenchlorid, Dimethylacetamid, Dimethyl formamid oder Acetonitril, gegebenenfalls im Gemisch mit Wasser. Die Temperatur liegt im allgemeinen zwischen -30°C und Raumtemperatur.

Gemäss Variante (b) des erfindungsgemässen Verfahrens wird eine Verbindung IV mit einer Verbindung V thioliert. Als allfällige Esterschutzgruppen kommen die gleichen in Betracht wie oben angegeben für die Verbindungen III. Abgangsgruppen Y sind beispielsweise Halogene, z.B. Jod, Acyloxyreste, z.B. niedere Alkanoyloxyreste, wie Acetoxy, niedere Alkyl- oder Arylsulfonyloxyreste, wie Mesyloxy oder Tosyloxy. Vorzugsweise wird Y in der Bedeutung Acetoxy eingesetzt. Dabei kann in an sich bekannter Weise vorgegangen werden, z.B. bei einer Temperatur zwischen etwa 40 und 80°C, zweckmässig bei etwa 60°C, in Wasser oder in einer Pufferlösung mit einem pH von etwa 6-7. Falls Y ein Halogen darstellt, soll M in Formel IV eine Esterschutzgruppe darstellen, wobei man vorzugsweise in einem polaren organischen Lösungsmittel, z.B. in Dimethylformamid, Essigester, Acetonitril oder Tetrahydrofuran, arbeitet. Die Temperatur liegt vorzugsweise zwischen -30°C und Raumtemperatur.

Gemäss Verfahrensvariante (c) des erfindungsgemässen Verfahrens wird ein Ketocephalosporin der Formel VI mit einem Salz eines O-substituierten Hydroxylamins der Formel VII umgesetzt. Als Salz kommt vorzugsweise ein Mineralsäuresalz, z.B. das Hydrochlorid, oder ein organisches Sulfonat, wie z.B. das p-Toluolsulfonat, in Betracht. Das Salz wird vorzugsweise in etwa äquimolarer Menge bis leichtem Ueberschuss eingesetzt. Die Umsetzung wird bevorzugt in einem polaren organischen Lösungsmittel durchgeführt, z.B. in Dimethylformamid, N-Methylpyrrolidon, Dimethylsulfoxid, Acetonitril, Wasser oder, insbesondere, in Dimethylacetamid. Bei Verwendung letzteren Lösungsmittels wird besonders hohe Anteile an syn-Form des Endproduktes erhalten. Die Temperatur liegt vorzugsweise zwischen 0°C und Raumtemperatur.

Allfällige Schutzgruppen einer erhaltenen Verbindung, z.B. an phenolischem Hydroxy oder Amino, können abgespalten werden; phenolische Hydroxyschutzgruppen z.B. wie folgt: Acetyl mit Wasser bei pH 7-8 oder mit Ammoniak, Trimethylsilyl mit Aethanol oder Wasser, Tetrahydropyranyl durch saure Hydrolyse z.B. mit wässriger Salzsäure. Aminoschutzgruppen können wie folgt abgespalten werden: Sauer abspaltbare Aminoschutzgruppen werden vorzugsweise mit Hilfe einer niederen Alkancarbonsäure, die gegebenenfalls halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereich von etwa 0°C bis 40°C. Alkalisch abspaltbare Schutzgruppen werden im allgemeinen mit verdünnter wässriger Lauge bei 0°C bis 30°C hydrolysiert. Die Chloracetyl-, Bromacetyl- und Jodacetylschutzgruppen können mittels Thioharnstoff in saurem, neutralem oder alkalischem Milieu bei etwa 0-30°C abgespalten werden.

Allfällige Carboxyschutzgruppen können wie folgt abgespalten werden: Wenn die Schutzgruppen eine Trimethylsilylgruppe darstellt, kann diese Gruppe besonders leicht durch Behandeln mit Wasser oder Aethanol entfernt werden. Benzhydryl- und t-Butylgruppen können mit Ameisensäure oder Trifluoressigsäure abgespalten werden. Die Temperatur ist in der Regel Raumtemperatur, obwohl auch leicht erhöhte bzw. leicht erniedrigte Temperatur angewendet werden kann, z.B. im Bereich von etwa 0°C bis 40°C. Allylgruppen entfernt man z.B. mittels Palladiumsalzen und tertiären Aminen, wie N-Methylpyrrolidin oder N-Methylmorpholin.

Zur Herstellung der leicht hydrolysierbaren Ester der Carbonsäuren der Formel I gemäss Variante (e) wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder Carbonat, oder einem organischen Amin, wie Triäthylamin, beschleunigt werden. Diese Reaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereich von etwa 0 bis 40°C.

Die Herstellung der Salze und Hydrate der Verbindungen der Formel I bzw. der Hydrate dieser Salze kann in an sich bekannter Weise erfolgen, z.B. durch Umsetzung der Carbonsäure der Formel I mit einer äquivalenten Menge der gewünschten Base, zweckmässig in einem Lösungsmittel wie Wasser, oder in einem organischen Lösungsmittel, wie Aethanol, Methanol, Aceton oder anderen mehr. Entsprechend wird Salzbildung durch Addition einer organischen oder anorganischen Säure herbeigeführt. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester oder Salze davon) einer feuchten Atmosphäre, z.B. bei etwa +10°C bis +40°C, ausgesetzt werden.

Die Ausgangsverbindungen der Formel III können durch Thiolierung in Analogie zur obigen Umsetzung der Verbindungen IV und V hergestellt werden. Gleiches gilt für die Herstellung der Ausgangsverbindungen der Formel VI.

Die Thiole der Formel V sind neue Verbindungen; entsprechende Carbamoylderivate (R¹ = eine Gruppe (a)-(d) können z.B. aus den entsprechenden Estern, z.B. dem 2-(Niederalkoxycarbonyl)-s-triazolo[1,5-a]pyrimidin-7-thiol (Eur. Pat. Publ. 150,507) oder dem 3-(Niederalkoxycarbonyl) -pyrazolo[1,5-a]pyrimidin-7-thiol (J. Med. Chem. 1981, 24(5), 610-13) bzw. aus analogen Estern hergestellt werden durch Aminolyse mit Ammoniak oder dem entsprechenden Amin; oder aus den entsprechenden Carbonsäuren in Gegenwart von 2-Halogenpyridiniumsalzen, z.B. von 2-Chlor- oder 2-Fluor-1-methylpyridiniumchlorid oder -tosylat, oder auch in Gegenwart von Dicyclohexylcarbodiimid, bevorzugt zusammen mit N-Hydroxybenztriazol oder N-Hydroxysuccinimid; oder auch aus dem entsprechenden Säurechlorid mit Ammoniak oder dem entsprechenden Amin. Die Herstellung entsprechender 5-Thiole erfolgt z.B. ausgehend von einem entsprechend substituierten 5-Amino-s-triazol, das mit einem ω,ω,ω-Trifluoracet- essigsäureester oder mit Diketen, gefolgt von einem Dehydratisierungsmittel, wie konz. Schwefelsäure oder Polyphosphorsäure, in das entsprechende 5-Hydroxy-s-triazole (bzw. pyrazolo)[1,5-a]pyrimidin übergeführt wird. Weiterverarbeitung zum gewünschten 5-Thiol erfolgt in Analogie zu der in J. Med. Chem. 1981, 24, 610-613 beschriebenen Synthese der 7-Thiole.

Ein allenfalls erhaltenes syn/anti-Gemisch einer Verbindung der Formel I kann in die entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches.

Die Verbindungen der Formel I sowie die entsprechenden leicht hydrolysierbaren Ester und Salze bzw. die Hydrate dieser Produkte sind antibiotisch, insbesondere bakterizid wirksam. Sie besitzen ein breites Wirkungsspektrum gegen Gram-positive und Gram-negative Mikroorganismen, einschliesslich Staphylokokken und verschiedene Cephalosporin-resistente Gram-negative Bakterien, wie z.B. Pseudomonas aeruginosa, Enterobacter cloacae, Escherichia coli, Serratia marcescens, Proteus- und Klebsiella-Spezies.

Die Verbindungen der Formel I sowie die entsprechenden leicht hydrolysierbaren Ester und Salze bzw. die Hydrate dieser Produkte können zur Behandlung und Prophylaxe von Infektionskrankheiten verwendet werden. Für den Erwachsenen kommt eine Tagesdosis von etwa 0,1 bis etwa 4 g in Betracht. Die parenterale Verabreichung der erfindungsgemässen Verbindungen ist besonders bevorzugt.

Zum Nachweis der antimikrobiellen Wirksamkeit der erwähnten Produkte wurden verschiedene der gemäss den nachstehenden Ausführungsbeispiele hergestellten Endprodukte auf ihre Aktivität in vitro getestet. Gemessen wurde die Mindesthemmkonzentration (µg/ml). Bezug wird genommen auf die Nummern der Ausführungsbeispiele:

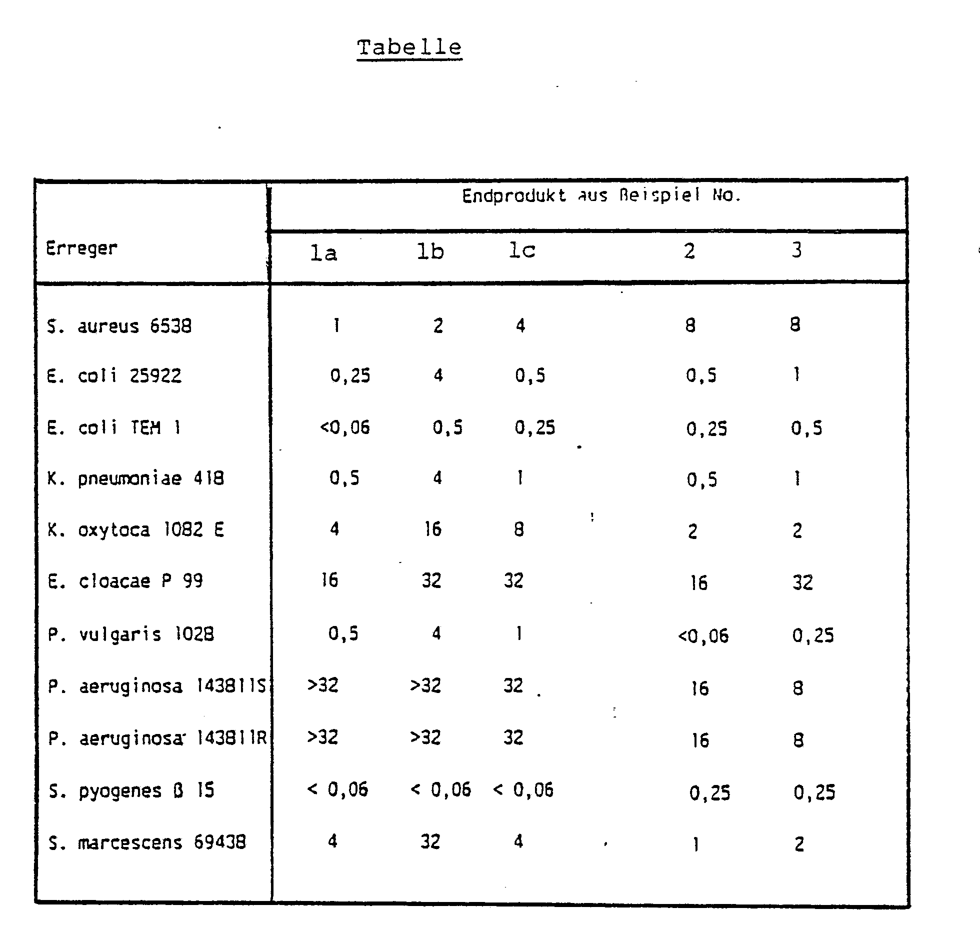

Die erfindungsgemässen Produkte können als Heilmittel, z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragees, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel. Salze zur Veränderung des osmotischen Druckes, Anaestetica oder Puffer. Sie können auch noch andere therapeutisch wertvolle Stoffe enthalten. Die Verbindungen der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien, wie Wasser oder isotonische Kochsalzlösung, zubereitet. Die leicht hydrolysierbaren Ester der Verbindungen der Formel I und ihre Salze bzw. Hydrate kommen auch für die enterale Verabreichung in Betracht.

In den nachstehenden Ausführungsbeispielen sind alle Temperaturangaben in Grad Celsius angegeben.

### Beispiel 1

a) 1,37 g (6R,7R)-7-Amino-3-[[(2-carbamoyl-5 -methyl-s-triazolo[1,5-a]pyrimidin-7-yl)thio]methyl]-8-oxo -5-thia-1-azabicyclo[4.2.0]oct-2-en-2-carbonsäure werden in 10 ml Methylenchlorid suspendiert und mit 2,44 ml Bistrimethylsilylacetamid versetzt. Nachdem alles in Lösung gegangen ist gibt man 1,16 g (2-(2-Amino-4-thiazolyl)-2-(Z)-methoxyimino-essigsäure -2-benzthiazolylthioester zu und rührt das Reaktions gemisch 1 Stunde bei Raumtemperatur. Man gibt 30 ml Methylenchlorid zu und fällt das Produkt durch Zutropfen von 2 ml Alkohol. Der Niederschlag wird abgenutscht, getrocknet und in 10 ml Dimethylformamid gelöst. Es werden 1,6 ml 2N wässriger Na-2-Aethylcaproat zugegeben und die entstandene Lösung in 150 ml Aether eingetropft. Das ausgefällte Produkt wird abgenutscht und getrocknet. Man erhält (6R,7R)-7-[(Z)-2-(2-Amino-4-thiazolyl) -2-(methoxyimino]acetamido]-3-[[[2-carbamoyl-5-methyl -s-triazolo[1,5-a]pyrimidin-7-yl]thio]-methyl]-8-oxo-5-thia -1-azabicyclo[4.2.0]oct-2-en-2-carbonsäure-Natriumsalz.
¹H NMR (DMSO-d₆): δ 2,60 (s,3H); 3,52 (d,J=18 Hz,1H); 3,73 (d,J=18 Hz,1H); 3,82 (s,3H); 4,42 (d,J=14 Hz,1H); 4,53 (d,J=14 Hz,1H); 5,13 (d,J=5 Hz,1H); 5,74 (dd,J=8 Hz und J= 5 Hz,1H); 6,73 (s,1H); 7,24 (s,2H); 7,56 (s,1H); 7,87 (s,1H); 8,17 (s,1H); 8.61 (d,J=8 Hz,1H) ppm
IR (KBr):1773.
MS (70 eV): 605 (M + H)⁺.

Die als Ausgangsverbindung verwendete (6R,7R)-7-Amino-3-[[(2-carbamoyl-5-methyl-s-triazolo[1,5-a]pyrimidin-7-yl) thio]methyl]―8-oxo-5-thia-1-azabicyclo[4.2.0]oct-2-en-2-carbonsäure kann wie folgt hergestellt werden:

Eine Suspension von 2,50 g 7-Mercapto-5-methyl-s-triazolo[1,5-a]pyrimidin -2-carbonsäure-methylester (Eur. Pat. Publ. 150,507) in 25 ml 25-proz. wässrigem Ammoniak wird 6 Stunden bei Raumtemperatur gerührt. Das Gemisch wird filtriert und der Feststoff im Vakuum bei 50° getrocknet. Man erhält 7-Mercapto-5-methyl-s-triazolo[1,5-a]pyrimidin -2-carbonsäureamid als 1:1:1 Addukt mit Wasser und Ammoniak.
¹H NMR (DMSO-d₆): δ 2,26 (s,3H); 6,72 (s,1H); 7,17 (s,4H,NH 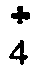 ); 7,60 (breites s, 1H); 7,84 (breites s, 1H) ppm.

Eine Mischung von 5,46 g (7R)-7-Aminocephalosporansäure und 4,85 g 7-Mercapto-5-methyl-s-triazolo[1,5-a]pyrimidin -2-carbonsäureamid-Ammoniumsalz wird unter gutem Rühren mit 50 ml einer 20-proz. Lösung von Bortrifluorid in Acetonitril versetzt. Die Temperatur wird mittels Eisbadkühlung auf unter 40° gehalten. Das Reaktionsgemisch wird 1 Stunde bei 20° gerührt und anschliessend mit 200 ml Wasser verdünnt. Es bildet sich ein weisser Niederschlag der durch Filtration gesammelt wird. Das noch feuchte Material wird in 50 ml 3N HCl gelöst und die Lösung filtriert. Aus dem Filtrat kristallisiert nach kurzer Zeit ein weisses Produkt aus. Durch Filtration, Waschen mit H₂O und Aceton und Trocknen im Vakuum erhält man (6R,7R)-7-Amino-3-[[(2-carbamoyl-5-methyl-s-triazolo[1,5-a]pyrimidin-7-yl)thio]methyl]-8--oxo-5-thia -1-azabicyclo[4.2.0]oct-2-en-2-carbonsäure als Hydrochlorid.
¹H NMR (DMSO-d₆): δ 2,61 (s,3H); 3,74 (d,J=17,5 Hz,1H); 2,87 (d,J=17,5 Hz,1H); 4,46 (d,J=12,5 Hz,1H); 4,54 (d,J=12,5 Hz,1H); 5,20 (d,J=5 Hz,1H); 5,25 (d,J=5 Hz,1H); 7,43 (s,1H); 7,89 (s,1H); 8,19 (s,1H) ppm.
MS: 442 (M + H)⁺.
IR (KBr): 1770.

In Analogie zur obigen Methode werden hergestellt:

b) (6R,7R)-7-[(Z)-2-(2-Amino-4-thiazolyl)-2-[(pivaloyloxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure-Natriumsalz.
¹H NMR (DMSO-d₆): δ 1,19 (s,9H); 2,58 (s,3H); 3,40 (d,J=18 Hz,1H); 3,61 (d,J=18 Hz,1H); 4,48 (d,J=14 Hz,1H); 4,66 (d,J=14 Hz,1H); 5,07 (d,J=5 Hz,1H); 5,65 (dd,J=8 Hz und J=5 Hz,1H); 6,98 (s,1H); 7,37 (s,2H); 7,86 (2s,2H); 8,17 (s,1H); 9,84 (d,J=8 Hz,1H) ppm.
IR (KBr): 1761.

c) (6R,7R)-7-[(Z)-2-(2-Amino-4-thiazolyl)-2-[(carbamoylmethoxy)imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure-Natriumsalz.
¹H NMR (DMSO-d₆): δ 2,59 (s,3H); 3,42 (d,J=18 Hz,1H); 3,62 (d,J=18 Hz,1H); 4,40 (s,2H); 4,42 (d,J=14 Hz,1H); 4,70 (d,J=14 Hz,1H); 5,05 (d,J=5 Hz,1H); 5,66 (dd,J=8 Hz und J=5 Hz,1H); 6,80 (s,1H); 7,12 (s,1H); 7,38 (s,2H); 7,50 (s,1H); 7,85 (s,2H); 8,24 (s,1H); 9,82 (d,J=8 Hz,1H) ppm.
IR (KBr): 1763.

d) (6R,7R)-7-[(Z)-2-(2-Amino-4-thiazolyl)-2-[[1-(t-butoxycarbonyl)-1-methyläthoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure-Natriumsalz.
¹H-NMR (DMSO-d₆): δ 1,38 (s,9H); 1,40 (s,3H); 1,42 (s,3H); 2,61 (s,3H); 3,58 (d,J= 18 Hz,1H); 3,80 (d,J=18 Hz,1H); 4,43 (m,2H); 5,20 (d,J=5 Hz,1H); 5,84 (dd,J=8 Hz und J=5 Hz,1H); 6,70 (s,1H); 7,27 (s,breit,2H); 7,42 (s,1H); 7,88 (s,1H); 8,17 (s,1H); 9,42 (d,J=8 Hz,1H) ppm.
IR (KBr): 1773
MS: 733 (M+H)⁺.

e) (6R,7R)-7-[(Z)-2-(2-Amino-4-thiazolyl)-2-[[(t-butoxycarbonyl)methoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure-Natriumsalz.
¹H-NMR (DMSO-d₆): δ 1,41 (s,9H); 2,61 (s,3H); 3,58 (d,J=18 Hz,1H); 3,81 (d,J=18 Hz,1H); 4,42 (m,2H); 4,54 (s,2H); 5,20 (d,J=5 Hz,1H); 5,82 (dd,J=8 Hz und J=5 Hz,1H); 6,78 (s,1H); 7,27 (s,breit,2H); 7,41 (s,1H); 7,88 (s,1H); 8,17 (s,1H); 9,57 (d,J=8 Hz,1H) ppm.
IR (KBr): 1771.
MS: 705 (M+H)⁺.

### Beispiel 2

740 mg (6R,7R)-7-[(Z)-2-(2-Amino-4-thiazolyl)-2-[[(t-butoxycarbonyl)methoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure-Natriumsalz werden in 5 ml Trifluoressigsäure gelöst und 1 Stunde bei Raumtemperatur gerührt. Das Reaktionsgemisch wird am Vakuum eingeengt und der Rückstand in Aethylacetat aufgenommen. Der entstandene weisse Niederschlag wird abfiltriert und getrocknet. Man erhält 680 mg (6R,7R)-7-[(Z)-2-(2-Amino-4-thiazolyl) -2-[(carboxymethoxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure-trifluoracetat als weisses Pulver.

### Beispiel 3

1,56 g (6R,7R)-7-[(Z)-2-(2-Amino-4-thiazolyl)-2-[[1-(t-butoxycarbonyl)-1-methyläthoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure werden in 10 ml Trifluoressigsäure gelöst und 1 Stunde bei Raumtemperatur gerührt. Das Reaktionsgemisch wird am Vakuum eingeengt und in Aethylacetat aufgenommen. Der erhaltene weisse Niederschlag wird abfiltriert und getrocknet. Man erhält (6R,7R)-7-[(Z)-2-(2-Amino-4-thiazolyl)-2-[1-(carboxy -1-methyläthoxy)imino]acetamido]-3-[[[2-carbamoyl-5-methyl -s-triazolo[1,5-a]pyrimidin-7-yl]thio]methyl]-8-oxo-5-thia -1-azabicyclo[4.2.0]oct-2-en-2-carbonsäure-trifluoracetat als weisses Pulver.

### Beispiel 4

0,228 g (0,4 mMol) (6R,7R)-7-(2-Amino-4-thiazolglyoxylamido) -3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure werden in 2 ml Dimethylacetamid gelöst. 0,140 g (0,52 mMol) 1-[2-(Aminooxy)-2-methylpropionyl]-2-[(1,4-dihydro-5-hydroxy-4-oxo-2-pyridyl)carbonyl]hydrazin und 0,100 g (0, 52 mMol) p-Toluolsulfonsäurehydrat werden zugegeben. Nach 20-stündigem Rühren bei Raumtemperatur wird das Dimethylacetamid am Hochvakuum abgedampft, der Rückstand in Wasser aufgenommen und zur vollständigen Fällung des Produkts 15 Minuten gerührt. Das Produkt wird abfiltriert, mit Wasser gut ausgewaschen und im Hochvakuum bei Raumtemperatur getrocknet. Man erhält (6R,7R)-7-[(Z)-2-(2-Amino-4-thiazolyl) -2-[[1-[3-[(1,4-dihydro-5-hydroxy-4-oxo-2-pyridyl)carbonyl]carbazoyl]-1-methyläthoxy]imino]acetamido] -3-[[[2-carbamoyl-5-methyl-s-triazolo[1,5-a]pyrimidin -7-yl(thio]methyl]-8-oxo-5-thia-1-azabicyclo[4.2.0]oct -2-en-2-carbonsäure als beiges Pulver.
¹H-NMR (DMSO-d₆): δ 1,46 (s,3H), 1,49 (s,3H), 2,61 (s,3H); 3,65 (d,J=18 Hz, 1H); 3,86 (d,J=18 Hz, 1H); 4,37 (d,J=14 Hz, 1H); 4,47 (d,J=14 Hz, 1H); 5,24 (d,J=5 Hz, 1H); 5,91 (dd,J=8 Hz und J=5 Hz, 1H); 6,90 (s,1H); 7,37 (s, 4H, breit); 7,99 (s,2H, breit); 8,19 (s,1H); 9,30 (s,1H); 9,65 (d,J=8 Hz, 1H).

Die als Ausgangsverbindung verwendete (6R,7R)-7-(2-Amino-4-thiazolglyoxylamido)-3-[[(2-carbamoyl -5-methyl-s-triazolo[1,5-a]pyrimidin-7-yl)thio]methyl] -8-oxo-5-thia-1-azabicyclo[4.2.0]oct-2-en-2-carbonsäure kann wie folgt hergestellt werden:

Eine Suspension von 2,50 g 7-Mercapto-5-methyl-s-triazolo[1,5-a]pyrimidin -2-carbonsäure-methylester (Eur. Pat. Publ. 150,507) in 25 ml 25-proz. wässrigen Ammoniak wird 6 Stunden bei Raumtemperatur gerührt. Das Gemisch wird filtriert und der Feststoff im Vakuum bei 50° getrocknet. Man erhält 7-Mercapto-5-methyl-s-triazolo[1,5-a]pyrimidin-2-carbonsäureamid als 1:1:1 Addukt mit Wasser und Ammoniak. ¹H NMR (DMSO-d₆): δ 2,26 (s,3H); 6,72 (s,1H); 7,17 (s,4H,NH 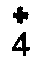 ); 7,60 (breites s, 1H); 7,84 (breites s, 1H) ppm.

Eine Mischung von 5,46 g (7R)-7-Aminocephalosporansäure und 4,85 g 7-Mercapto-5-methyl-s-triazolo[1,5-a]pyrimidin-2-carbonsäureamid-Ammoniumsalz wird unter gutem Rühren mit 50 ml einer 20-proz. Lösung von Bortrifluorid in Acetonitril versetzt. Die Temperatur wird mittels Eisbadkühlung auf unter 40° gehalten. Das Reaktionsgemisch wird 1 Stunde bei 20° gerührt und anschliessend mit 200 ml Wasser verdünnt. Es bildet sich ein weisser Niederschlag der durch Filtration gesammelt wird. Das noch feuchte Material wird in 50 ml 3N HCl gelöst und die Lösung filtriert. Aus dem Filtrat kristallisiert nach kurzer Zeit ein weisses Produkt aus. Durch Filtration, Waschem mit H₂O und Aceton und Trocknen im Vakuum erhält man (6R,7R)-7-Amino-3-[[(2-carbamoyl-5-methyl-s-triazolo[1,5-a]pyrimidin-7-yl)thio]methyl]-8-oxo-5-thia -1-azabicyclo[4.2.0]oct-2-en-2-carbonsäure als Hydrochlorid.
¹H NMR (DMSO-d₆): δ 2,61 (s,3H); 3,74 (d,J=17,5 Hz,1H); 2,87 (d,J=17,5 Hz,1H); 4,46 (d,J=12,5 Hz,1H); 4,54 (d,J=12,5 Hz,1H); 5,20 (d,J=5 Hz,1H); 5,25 (d,J=5 Hz,1H); 7,43 (s,1H); 7,89 (s,1H); 8,19 (s,1H) ppm.
MS: 422 (M + H)⁺.
IR (KBr): 1770.

Eine Suspension von 1,71 g (6R,7R)-7-Amino-3-[[(2-carbamoyl-5-methyl -s-triazolo[1,5-a]pyrimidin-7-yl)thio]methyl]-8-oxo-5-thia -1-azabicyclo[4.2.0]oct-2-en-2-carbonsäure-hydrochlorid in 20 ml Methylenchlorid wird mit 2,74 ml N,O-Bis-(trimethylsilyl)acetamid versetzt. Nachdem alles in Lösung gegangen ist, werden 1,32 g 2-Amino-4-thiazolthioglyoxylsäure-S-(2-benzothiazolyl)ester zugesetzt und das Gemisch 1,5 Stunden bei 20° gerührt. Ungelöstes Material wird durch Filtration abgetrennt und das Filtrat wird mit 40 ml Methylenchlorid verdünnt. Beim Zutropfen von 2 ml Aethanol entsteht ein gelber Niederschlag, welcher durch Filtration gesammelt und im Vakuum getrocknet wird. Man erhält (6R,7R)-7-(2-Amino-4-thiazolglyoxylamido)-3-[[(2-carbamoyl-5-methyl-s-triazolo[1,5-a]pyrimidin 7-yl)thio]methyl]-8-oxo-5-thia-1-azabicyclo[4.2.0]oct-2-en-2-carbonsäure als gelbes Pulver.
¹H NMR (DMSO-d₆): δ 2,60 (s,3H); 3,54 (d,J=17,5 Hz,1H); 3,74 (d,J=17,5 Hz,1H); 4,42 (d,J=14 Hz,1H); 4,56 (d,J=14 Hz,1H); 5,14 (d,J=5 Hz,1H); 5,72 (d,J=5 Hz,1H); 7,41 (s,2H); 7,55 (s,1H) ppm.

Das als Ausgangsverbindung verwendete 1-[2-(Aminooxy)-2-methylpropionyl]-2-[(1,4-dihydro -5-hydroxy-4-oxo-2-pyridyl)carbonyl]hydrazin wird wie folgt hergestellt:
1,20 g (7,1 mMol) (1,4-Dihydro-5-hydroxy-4-oxo-2-pyridyl)carbonylhydrazin werden in 120 ml Acetonitril suspendiert. Nach Zugabe von 2,88 g (14,1 mMol) Bis(trimethylsilyl)acetamid rührt man 2 Stunden bei 90°. Zu der auf Raumtemperatur abgekühlten Lösung werden 2,83 g (7,1 mMol) 2-Methyl-2-(phthalimidooxy)propionsäure-2-benzothiazol-thiolester gegeben. Nach 20-stündigem Rühren wird die Lösung im Vakuum bei Raumtemperatur eingeengt. Der Rückstand wird in 180 ml Aethanol aufgenommen und 7 Minuten unter Rückfluss gekocht. Ungelöstes Material wird abfiltriert und das Filtrat konzentriert. Durch Zugabe von Aether wird das Produkt kristallisiert. Man erhält 1-[(1,4-Dihydro-5-hydroxy -4-oxo-2-pyridyl)carbonyl]-2-[2-methyl -2-(phthalimidooxy)propionyl]hydrazin als weisse Kristalle vom Schmelzpunkt 239-241°.

0,670 g (1,675 mMol) 1-[(1,4-Dihydro-5-hydroxy-4-oxo-2-pyridyl)carbonyl]-2-[2-methyl-2-(phthalimidooxy)propionyl]hydrazin werden in 1,5 ml Dimethylformamid gelöst und bei 0° mit 0,077 g (1,675 mMol) Methylhydrazin versetzt. Nach 2 1/2-stündigem Rühren bei Raumtemperatur werden etwa 2 ml Aethanol zugegeben. Die dabei entstandenen Kristalle werden abfiltriert und verworfen. Das Filtrat wird im Hochvakuum eingedampft und das Produkt aus Aethanol kristallisiert. Man erhält 1-[2-(Aminooxy)-2-methylpropionyl] -2-[(1,4-dihydro-5-hydroxy-4-oxo-2-pyridyl)carbonyl]hydrazin als weisse Kristalle vom Schmelzpunkt 234° (Zersetzung).

### Beispiel 5

0,230 g (0,4 mMol) (6R,7R)-7-(2-Amino-4-thiazolglyoxylamido) -3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure werden in 3,5 ml Dimethylacetamid gelöst. Innerhalb von 6 Stunden werden 0,11 g (0,7 mMol) 2-(Aminooxy)methyl-5-hydroxy-4(1H)-pyrimidinon und 0,133 g (0,7 mMol) p-Toluolsulfonsäurehydrat zugegeben. Nach weiterem 18-stündigem Rühren wird das Dimethylacetamid im Hochvakuum abgedampft, der Rückstand in Wasser aufgenommen und zur vollständigen Fällung des Produktes 20 Minuten gerührt. Das Produkt wird filtriert, mit Wasser gut ausgewaschen und im Hochvakuum bei Raumtemperatur getrocknet. Man erhält (6R,7R)-7-[(Z)-2-(2-Amino-4-thiazolyl) -2-[[(1,4-dihydro-5-hydroxy-4-oxo -2-pyrimidinyl)methoxy]imino]acetamido] -3-[[(2-carbamoyl-5-methyl-s-triazolo[1,5-a]pyrimidin -7-yl)thio]-methyl]-8-oxo-5-thia-1-azabicyclo[4.2.0]oct -2-en-2-carbonsäure als beiges Pulver.
¹H-NMR (DMSO-d₆): δ 2,61 (s,3H); 3,53 (d,J=18 Hz,1H); 3,82 (d,J=18 Hz,1H); 4,38 (d,J=14 Hz,1H); 4,46 (d,J=14 Hz,1H); 4,92 (s, breit, 2H); 5,20 (d,J=5 Hz, 1H); 5,86 (dd,J=8 Hz und J=5 Hz,1H); 6,87 (s,1H); 7,38 (s,1H); 7,39 (s, breit, 3H); 7,88 (s,1H); 8,18 (s,1H); 9,93 (d,J=8 Hz,1H).

Das als Ausgangsverbindung verwendete 2-(Aminooxy)methyl-5-hydroxy-4(1H)-pyrimidinon wird wie folgt hergestellt:

0,696 g (3 mMol) 5-Hydroxy-2-hydroxymethyl-4(1H)-pyrimidinon, 0,787 g (3 mMol) Triphenylphosphin und 0,489 g (3 mMol) N-Hydroxyphthalimid werden in 50 ml Dimethylacetamid gelöst. Bei 20° wird eine Lösung von 0,640 g (3,3 mMol) Azodicarbonsäurediäthylester in 2 ml Dimethylacetamid zugetropft. Man rührt 20 Stunden bei Raumtemperatur, dampft das Lösungsmittel im Hochvakuum ab und kristallisiert das verbleibende gelbe Oel aus Aethanol/Aether. Nach dem Umkristallisieren aus Aethanol erhält man 4-[(5-Benzyloxy)-1,4-dihydro-4-oxo-2-pyrimidinyl]methoxy]phthalimid als weisse Kristalle vom Schmelzpunkt 157°.

0,754 g (2 mMol) N-[(5-Benzyloxy)-1,4-dihydro-4-oxo-2-pyrimidinyl]methoxy] phthalimid werden in 60 ml Methylenchlorid suspendiert. Bei -70° werden 560 mg (2,2 mMol) Bortribromid zugetropft. Man rührt 4 Stunden bei -70°, lässt auf Raumtemperatur erwärmen und rührt 16 Stunden weiter. Das Lösungsmittel wird im Vakuum abgedampft, der Rückstand mit 30 ml Wasser versetzt und gerührt. Das Produkt wird filtriert, mit Wasser und Aether gewaschen und im Hochvakuum bei 35° getrocknet. Man erhält 5-Hydroxy-2-[(phthalimidooxy)methyl]-4(1H)-pyrimidinon als weisse Kristalle vom Schmelzpunkt 174-175°.

0,875 g (3 mMol) 5-Hydroxy-2[(phthalimidooxy)methyl]-4(1H)-pyrimidinon werden in 7 ml Dimethylformamid suspendiert. Bei 10° gibt man 136 mg (3 mMol) Methylhydrazin dazu und rührt 1 Stunde bei Raumtemperatur. Die Lösung wird viermal mit 50 ml n-Pentan gewaschen, der feste Rückstand in Aether aufgenommen, das Produkt filtriert und aus Aethanol kristallisiert. Man erhält 2-(Aminooxy)methyl-5-hydroxy-4(1H)-pyrimidinon als weisse Kristalle vom Schmelzpunkt 163° (Zers.).

### Beispiel 6

### Herstellung von Trockenampullen für die intramuskuläre Verabreichung:

Es wird in üblicher Weise ein Lyophilisat von 1 g des Natriumsalzes der (6R,7R)-7-[(Z)-2-(2-Amino-4-thiazolyl)-2-(methoxyimino]acetamido]-3-[[[2-carbamoyl-5-methyl-s-triazolo[1,5-a]pyrimidin-7-yl]thio]-methyl]-8-oxo-5-thia― 1-azabicyclo[4.2.0]oct-2-en-2-carbonsäure herstellt und in eine Ampulle abgefüllt. Vor der Verabreichung wird das Lyophilisat mit 2,5 ml einer 2%-igen wässrigen Lidokainhydrochloridlösung versetzt.

## Patentansprüche

1. Acylderivate der allgemeinen Formel 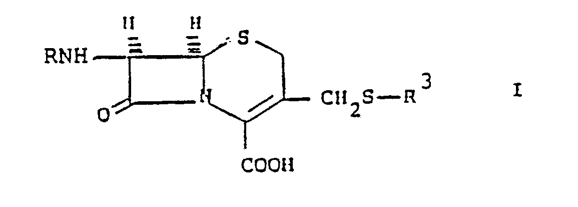 in der R einen Acylrest darstellt,
ausgenommen den Acylrest der allgemeinen Formel 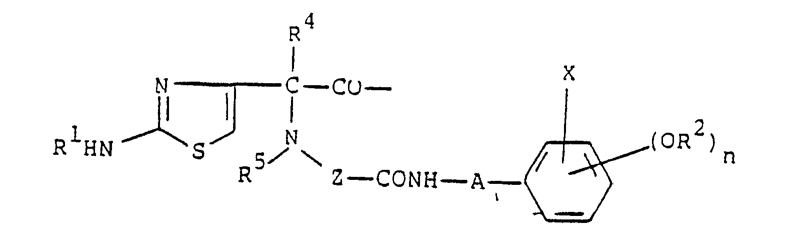 in der A eine Gruppe der allgemeinen Formeln
-NHCO-, -NHCONHCO-, -NHCOCH=CH-,-N= 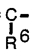
bedeutet, worin R⁶ Wasserstoff oder niederes Alkyl darstellt; R¹ und R² jeweils Wasserstoff oder eine Schutzgruppe, X Wasserstoff, Halogen, niederes Alkoxy, Nitro oder eine zusätzliche Gruppe -OR², n die Zahl 1 oder 2, R⁴ und R⁵ beide Wasserstoff oder zusammen eine zusätzliche Bindung und Z eine direkte Bindung oder Carbonyl (falls R⁴ und R⁵ beide Wasserstoff darstellen) oder eine Gruppe der Formel -O-B- (falls R⁴ und R⁵ zusammen eine zusätzliche Bindung darstellen) bedeutet, worin B geradkettiges, verzweigtes oder cyclisches niederes Alkylen bedeutet;
und worin ferner R³ eine substituierte bicyclische Gruppe der allgemeinen Formeln 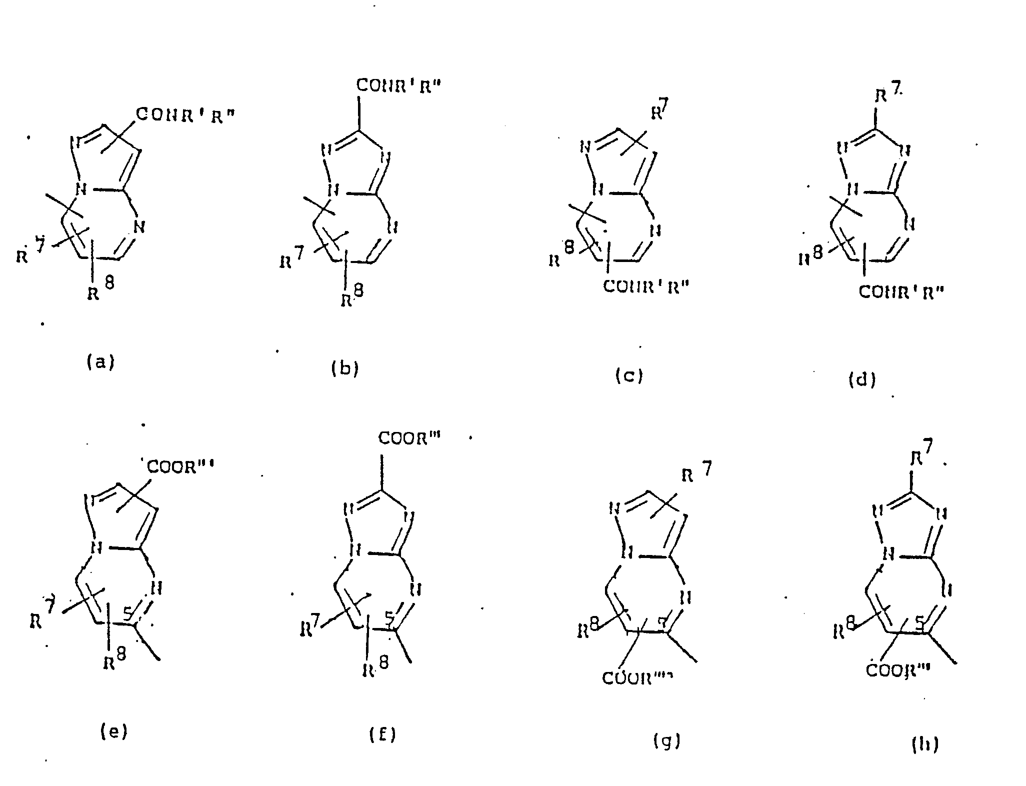 bedeutet, worin R⁷ und R⁸ je einzeln Wasserstoff, niederes Alkyl oder Trifluormethyl oder (in den Formeln (a), (b), (e) und (f)) zusammen Alkylen mit 3 oder 4 Kohlenstoffatomen darstellen, und Rʹ, Rʺ und R‴ je einzeln Wasserstoff, niederes Alkyl oder niederes Cycloalkyl bedeuten; wobei Verbindungen der Formel I, worin R₄ und R₅ zusammen eine zusätzliche Bindung darstellen, in der syn-isomeren Form vorliegen bzw. als Gemisch mit der anti-isomeren Form, worin die syn-isomere Form überwiegt, sowie leicht hydrolysierbare Ester und Salze dieser Verbindungen und Hydrate der Verbindungen der Formel I bzw. von deren Estern und Salzen.

2. Acylderivate nach Anspruch 1 der allgemeinen Formel 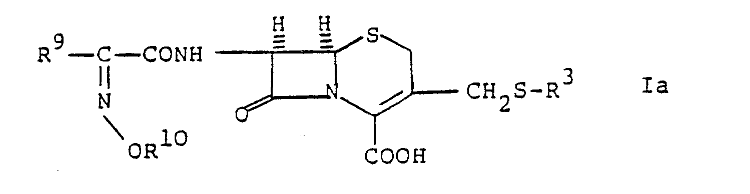 in der R³ die obige Bedeutung hat, R⁹ einen gegebenenfalls substituierten 5- oder 6-gliedrigen Heterocyclus mit 1-4, vorzugsweise 1 oder 2 Heteroatomen, wie N, O, und/oder S, darstellt und R¹⁰ Wasserstoff, niederes Alkanoyl, niederes Alkyl , niederes Alkenyl, niederes Cycloalkyl, niederes Cycloalkenyl oder niederes Alkyl substituiert durch Carboxy, Carbamoyl, niederes Alkoxycarbonyl oder niederes Alkanoyl bedeutet, oder R¹⁰ einen Rest der allgemeinen Formeln
-P-Q
-P-CO-Q
-P-CO-NH-NH-CO-Q¹
bedeutet, worin P niederes Alkylen oder niederes Cycloalkylen bedeutet, Q eine der Gruppen 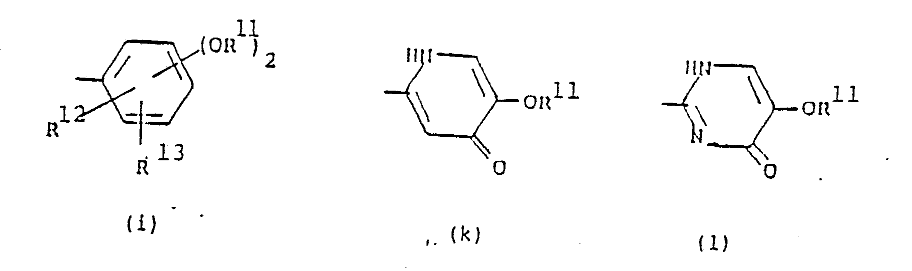 darstellt, worin R¹¹ Wasserstoff, niederes Alkanoyl oder tri(niederes Alkyl)-Silyl, R¹² Wasserstoff, -OR¹¹, niederes Alkoxy, Halogen, -OCOR¹⁴,-OCOOR¹⁴, -N(R¹⁴)₂, -NH-COR¹⁴, -NHCOOR¹⁴, -COR¹⁴, -SR¹⁴, -SOR¹⁴, -SO₂R¹⁴, -SO₃H, -COOR¹⁴, -CON(R¹⁴)₂ oder Nitro, R¹³ Wasserstoff oder Halogen und R¹⁴ Wasserstoff oder niederes Alkyl bedeutet, wobei in der Gruppe (i) die beiden Reste -OR¹¹ in 2,3- oder 3,4-Stellung stehen; und Q¹ eine der letztgenannten Gruppen (k) und (l) bedeutet, wobei die Verbindungen der Formel Ia in der syn-isomeren Form vorliegen bzw. als Gemisch mit der anti-isomeren Form, worin die syn-isomere Form überwiegt,
sowie leicht hydrolysierbare Ester und Salze dieser Verbindungen und Hydrate der Verbindungen der Formel Ia bzw. von deren Estern und Salzen.

3. Verbindungen nach Anspruch 1 oder 2,dadurch gekennzeichnet, dass R³ eine der Gruppen 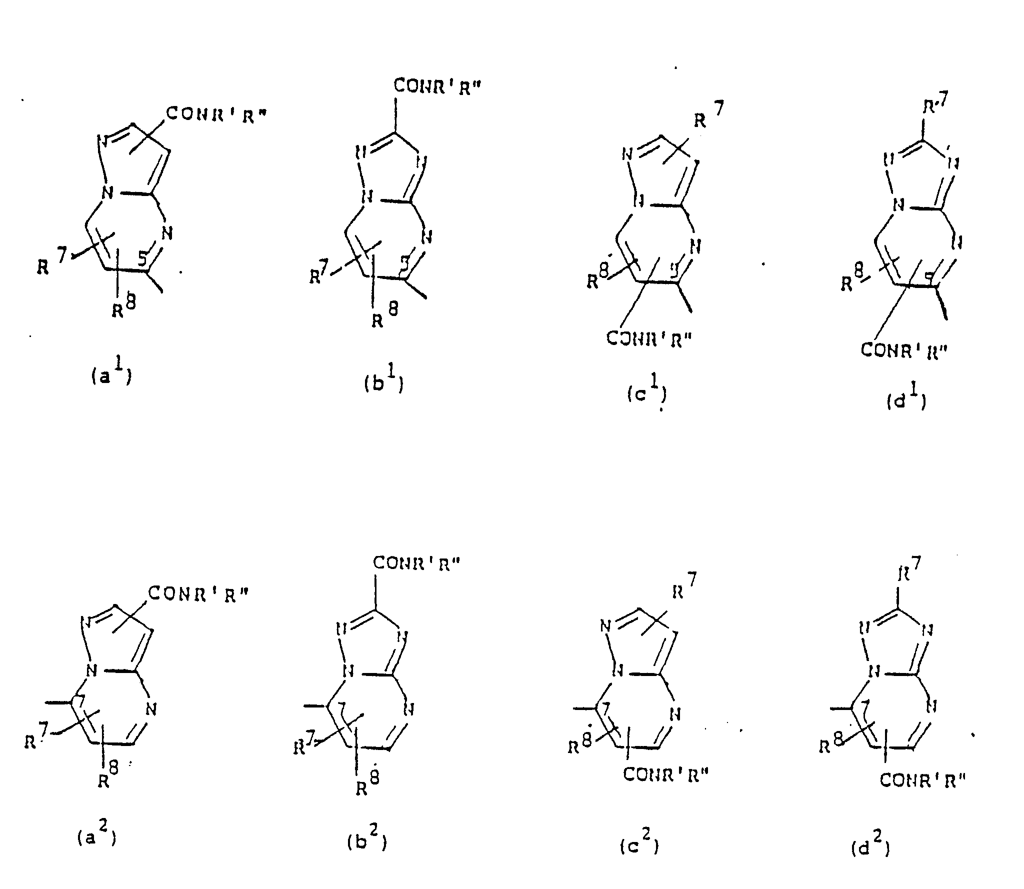 darstellt, worin Rʹ, Rʺ, R⁷ und R⁸ die in Anspruch 1 gegebene Bedeutung haben.

4. Verbindungen nach Anspruch 1 oder 2,dadurch gekennzeichnet, dass R³ eine Gruppe 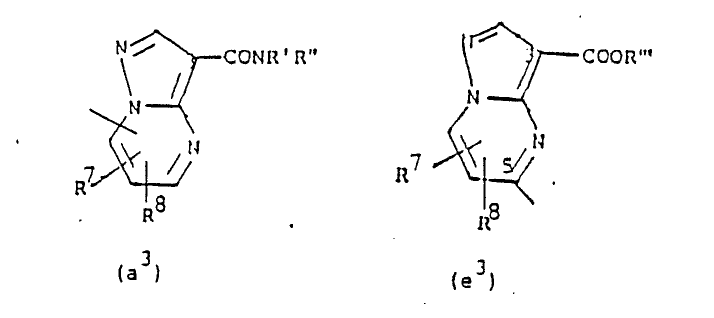 darstellt, worin Rʹ, Rʺ, und R⁷ und R⁸ die in Anspruch 1 gegebene Bedeutung haben.

5. Verbindungen nach Anspruch 1 oder 2, dadurch gekennzeichnet, dass R³ eine carbamoylsubstituierte bicyclische Gruppe der allgemeinen Formeln 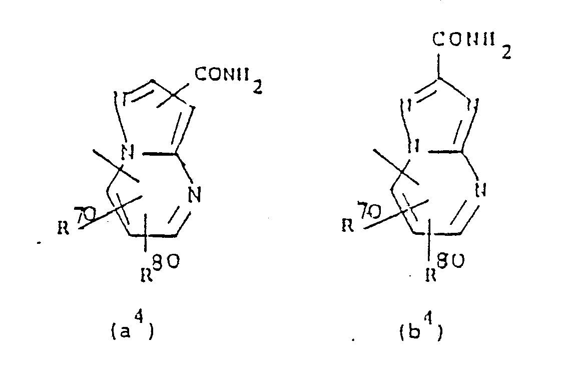 bedeutet, worin R⁷⁰ und R⁸⁰ je einzeln Wasserstoff, Methyl oder Trifluormethyl oder zusammen Alkylen mit 3 oder 4 Kohlenstoffatomen darstellen.

6. Verbindungen nach Anspruch 1 oder 2, dadurch gekennzeichnet, dass R³ eine substituierte bicyclische Gruppe der allgemeinen Formeln 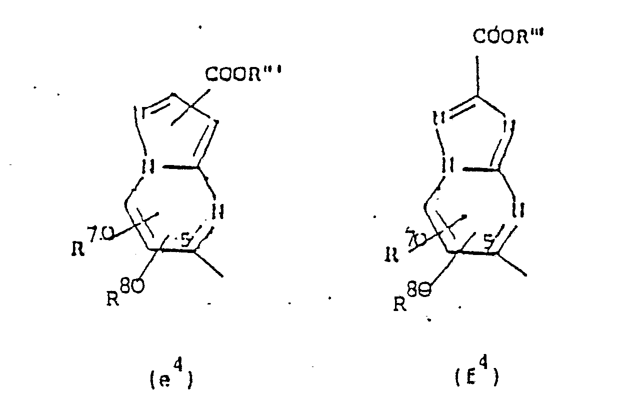 bedeutet, worin R⁷⁰ und R⁸⁰ je einzeln Wasserstoff, Methyl oder Trifluormethyl oder zusammen Alkylen mit 3 oder 4 Kohlenstoffatomen darstellen und R‴ Wasserstoff oder Methyl bedeutet.

7. Verbindungen nach Anspruch 1 oder 2, dadurch gekennzeichnet, dass R³ eine der Gruppen 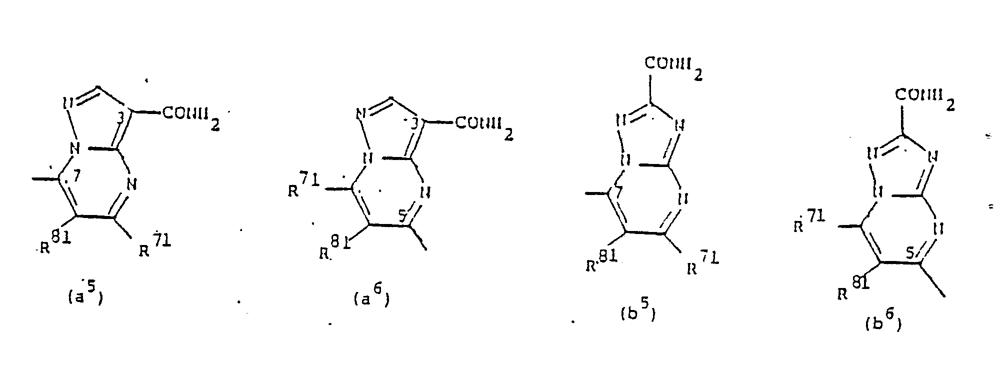 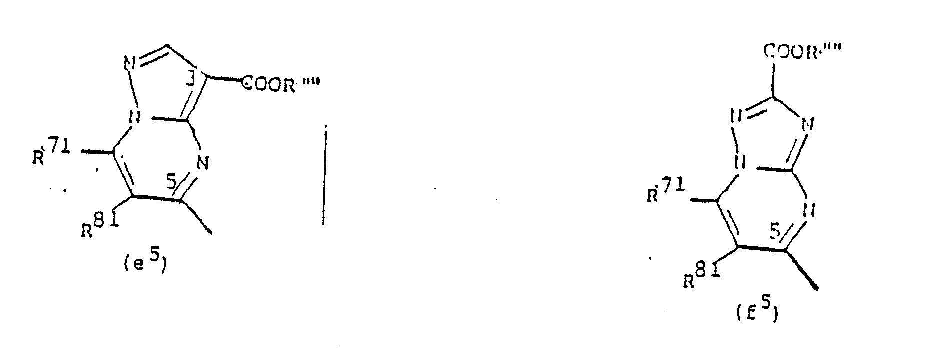 darstellt,
worin R⁷¹ Methyl oder Trifluormethyl und R⁸¹ Wasserstoff oder R⁷¹ und R⁸¹ zusammen Tetramethylen darstellen und Rʺʺ Wasserstoff oder Methyl bedeutet.

8. Verbindungen nach Anspruch 5, dadurch gekennzeichnet, dass R³ die Gruppe (a⁴) darstellt, welche über die Stellung 5 verknüpft ist:

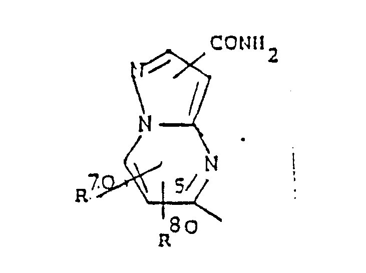

9. Verbindungen nach Anspruch 5, dadurch gekennzeichnet, dass R³ die Gruppe (a⁴) darstellt, welche über die Stellung 7 verknüpft ist:

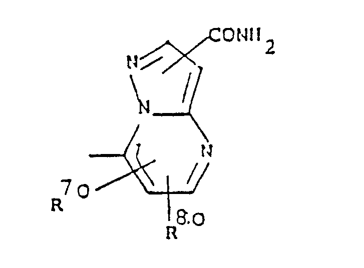

10. Verbindungen nach einem derAnsprüche 5, 8 und 9, dadurch gekennzeichnet, dass R³ die Gruppe (a⁴) darstellt, worin die Carbamoylgruppe in Stellung 3 steht:

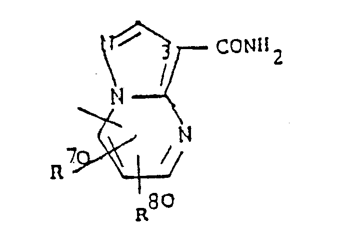

11. Verbindungen nach Anspruch 9 oder 10, dadurch gekennzeichnet, dass R³ die Gruppe 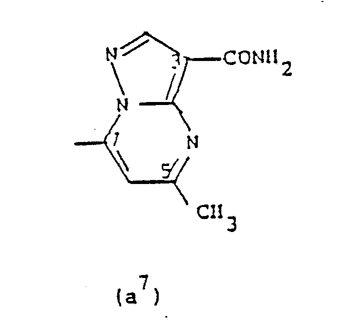 darstellt.

12. Verbindungen nach Anspruch 5, dadurch gekennzeichnet, dass R³ die Gruppe (b⁴) darstellt, welche über die Stellung 5 verknüpft ist:

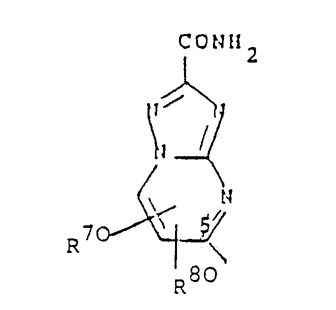

13. Verbindungen nach Anspruch 5,. dadurch gekennzeichnet, dass R³ die Gruppe (b⁴) darstellt, welche über die Stellung 7 verknüpft ist:

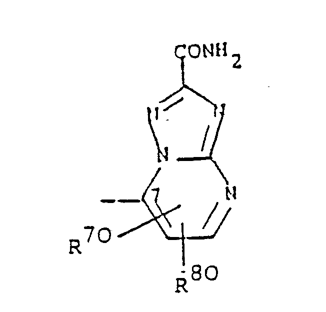

14. Verbindungen nach Anspruch 12, oder 13, dadurch gekennzeichnet, dass R³ die Gruppe 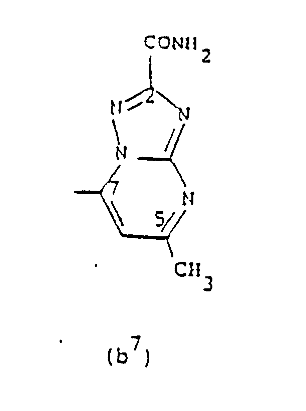 darstellt.

15. Verbindungen nach einem der Ansprüche 2-14, dadurch gekennzeichnet, dass R⁹ 2-Amino-4-thiazolyl oder 5-Amino-3-thiadiazolyl darstellt.

16. Verbindungen nach einem der Ansprüche 2-15, dadurch gekennzeichnet, dass R¹⁰ Wasserstoff, niederes Alkanoyl, niederes Alkyl oder niederes Alkyl substituiert durch Carboxy, Carbamoyl, niederes Alkoxycarbonyl oder niederes Alkanoyl darstellt.

17. Verbindungen nach Anspruch 16, dadurch gekennzeichnet, dass R¹⁰ Methyl, Allyl, Carboxymethyl, Carbamoylmethyl, 1-Carboxy-1-methyläthyl oder Pivaloyl darstellt.

18. Verbindungen nach einem der Ansprüche 2-15, dadurch gekennzeichnet, dass R¹⁰ eine der Gruppen
-P¹-Q
-P¹-CO-Q
-P¹-CO-NH-NH-CO-Q¹
worin Q und Q¹ die in Anspruch 2 gegebene Bedeutung haben und P¹ die Gruppe -CH₂- oder 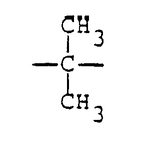 bedeutet,
darstellt.

19. Verbindungen nach Anspruch 18, dadurch gekennzeichnet, dass R¹⁰ eine der Gruppen 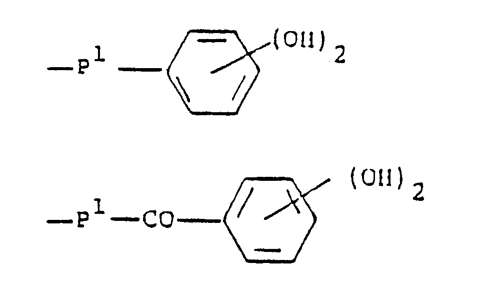 worin P¹ die in Anspruch 18 gegebene Bedeutung hat und die beiden Hydroxygruppen in 2,3- oder 3,4-Stellung stehen,
darstellt.

20. Verbindungen nach Anspruch 18, dadurch gekennzeichnet, dass R¹⁰ eine der Gruppen 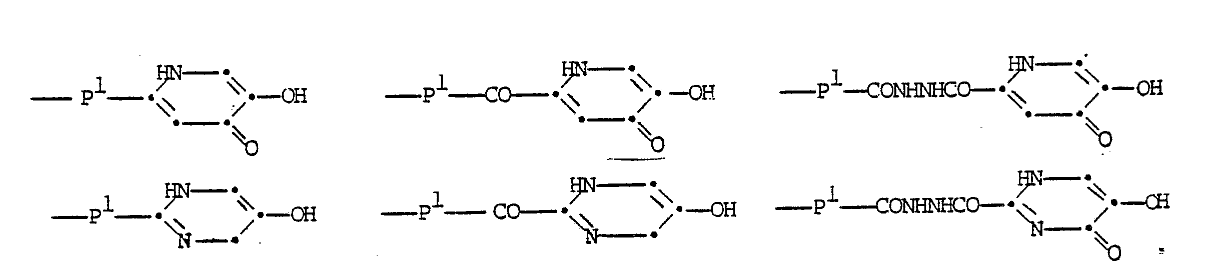 worin P¹ die in Anspruch 18 gegebene Bedeutung hat, darstellt.

21. (6R,7R)-7-[(Z)-2-(2-Amino-4-thiazolyl)-2-(methoxyimino]-acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[(t-butoxycarbonyl)methoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[(pivaloyloxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]-oct-2-en-2-carbonsäure,
(6R,7R)-7-[(Z)-2-(2-Amino-4-thiazolyl)-2-[[1-(t-butoxycarbonyl)-1-methyläthoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[(carboxymethoxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[1-(carboxy-1-methyläthoxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[(carbamoylmethoxy)imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure sowie Salze dieser Verbindungen.

22. (6R,7R)-7-[(Z)-2-(2-Amino-4-thiazolyl)-2-[[1-[3-[(1,4-dihydro-5-hydroxy-4-oxo-2-pyridyl)carbonyl]carbazoyl]-1-methyläthoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[(1,4-dihydro-5-hydroxy-4-oxo-2-pyrimidinyl)methoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure sowie Salze dieser Verbindungen.

23. Verbindungen der allgemeinen Formel 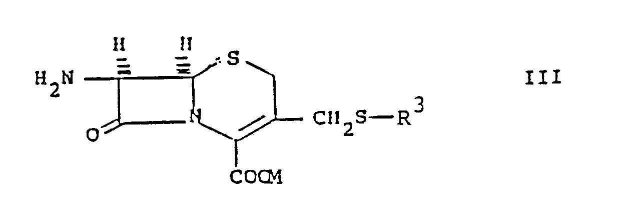 in der R³ die in Anspruch 1 gegebene Bedeutung hat und M Wasserstoff oder eine Esterschutzgruppe darstellt.

24. Verbindungen der allgemeinen Formel
HSR³ V
in der R³ die in Anspruch 1 gegebene Bedeutung hat.

25. Verbindungen der allgemeinen Formel 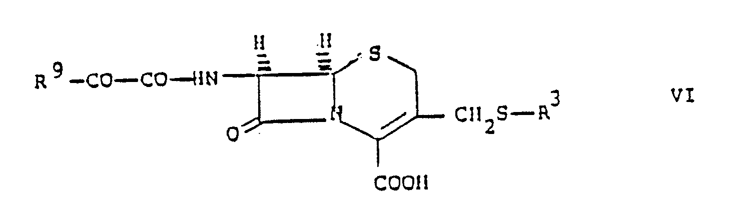 worin R³ die in Anspruch 1 gegebene Bedeutung und R⁹ die in Anspruch 2 gegebene Bedeutung hat.

26. Verbindungen nach einem der Ansprüche 1-22 als pharmazeutische Wirkstoffe.

27. Verbindungen nach einem der Ansprüche 1-22 als pharmazeutische Wirkstoffe zur Behandlung und Prophylaxe von Infektionskrankheiten.

28. Verfahren zur Herstellung der Verbindungen gemäss einem der Ansprüche 1-22, dadurch gekennzeichnet, dass man
(a) eine Verbindung der allgemeinen Formel 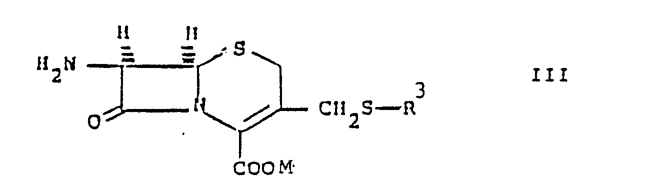 in der R³ die in Anspruch 1 gegebene Bedeutung hat und M Wasserstoff oder eine Esterschutzgruppe darstellt, mit einem Acylierungsmittel umsetzt und allfällige Schutzgruppe gewünschtenfalls abspaltet, oder dass man
(b) eine Verbindung der allgemeinen Formel 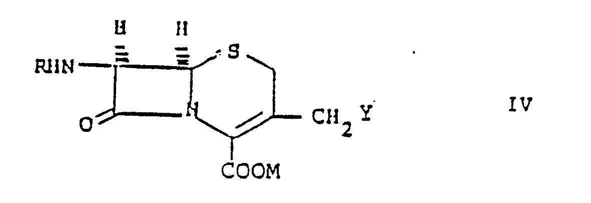 in der R und M die in Anspruch 1 gegebene Bedeutung haben und Y eine Abgangsgruppe darstellt,
mit einer Verbindung der allgemeinen Formel
HSR³ V
in der R³ die in Anspruch 1 gegebene Bedeutung hat,
umsetzt und allfällige Schutzgruppen gewünschtenfalls abspaltet, oder dass man
(c) zur Herstellung von Verbindungen der Formel I, worin R die allgemeine Formel 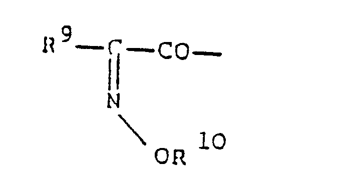 worin R⁹ und R¹⁰ die in Anspruch 2 gegebene Bedeutung haben,
hat, eine Verbindung der allgemeinen Formel 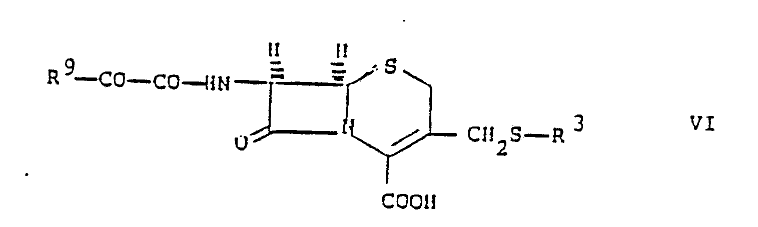 worin R³ die in Anspruch 1 und R⁹ die in Anspruch 2 gegebene Bedeutung haben,
mit einem Salz einer Verbindung der allgemeinen Formel
NH₂OR¹⁰ VII
worin R¹⁰ die in Anspruch 2 gegebene Bedeutung hat, umsetzt, oder dass man
(d) zur Herstellung eines leicht hydrolysierbaren Esters einer Verbindung der Formel I eine Carbonsäure der Formel I einer entsprechenden Veresterung unterwirft, oder dass man
(e) zur Herstellung von Salzen und Hydraten eine Verbindung der Formel I bzw. Hydraten dieser Salze eine Verbindung der Formel I in ein Salz oder Hydrat bzw. ein Hydrat dieses Salzes überführt.

29. Pharmazeutische Präparate, gekennzeichnet durch einen Gehalt an einer Verbindung gemäss einem der Ansprüche 1-22.

30. Pharmazeutische Präparate zur Behandlung und Prophylaxe von Infektionskrankheiten, gekennzeichnet durch einen Gehalt an einer Verbindung gemäss einem der Ansprüche 1-22.

31. Verwendung von Verbindungen gemäss einem der Ansprüche 1-22 bei der Behandlung bzw. Prophylaxe von Krankheiten.

32. Verwendung von Verbindungen gemäss einem der Ansprüche 1-22 bei der Behandlung bzw. Prophylaxe von Infektionskrankheiten.

33. Verwendung von Verbindungen gemäss einem der Ansprüche 1-22 bei der Herstellung von Arzneimittlen für die Behandlung bzw. Prophylaxe von Infektionskrankheiten.

## Patentansprüche (Patentansprüche für folgende(n) Vertragsstaat(en): ES, GR)

1. Verfahren zur Herstellung von Acylderivaten der allgemeinen Formel 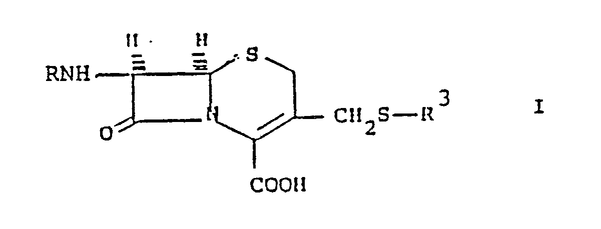 in der R einen Acylrest darstellt,
ausgenommen den Acylrest der allgemeinen Formel 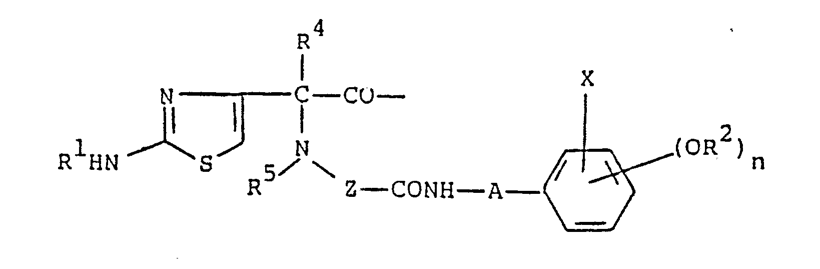 in der A eine Gruppe der allgemeinen Formeln
-NHCO-, -NHCONHCO-, -NHCOCH=CH-,-N= 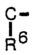
bedeutet, worin R⁶ Wasserstoff oder niederes Alkyl darstellt; R¹ und R² jeweils Wasserstoff oder eine Schutzgruppe, X Wasserstoff, Halogen, niederes Alkoxy, Nitro oder eine zusätzliche Gruppe -OR², n die Zahl 1 oder 2, R⁴ und R⁵ beide Wasserstoff oder zusammen eine zusätzliche Bindung und Z eine direkte Bindung oder Carbonyl (falls R⁴ und R⁵ beide Wasserstoff darstellen) oder eine Gruppe der Formel -O-B- (falls R⁴ und R⁵ zusammen eine zusätzliche Bindung darstellen) bedeutet, worin B geradkettiges, verzweigtes oder cyclisches niederes Alkylen bedeutet;
und worin ferner R³ eine substituierte bicyclische Gruppe der allgemeinen Formeln 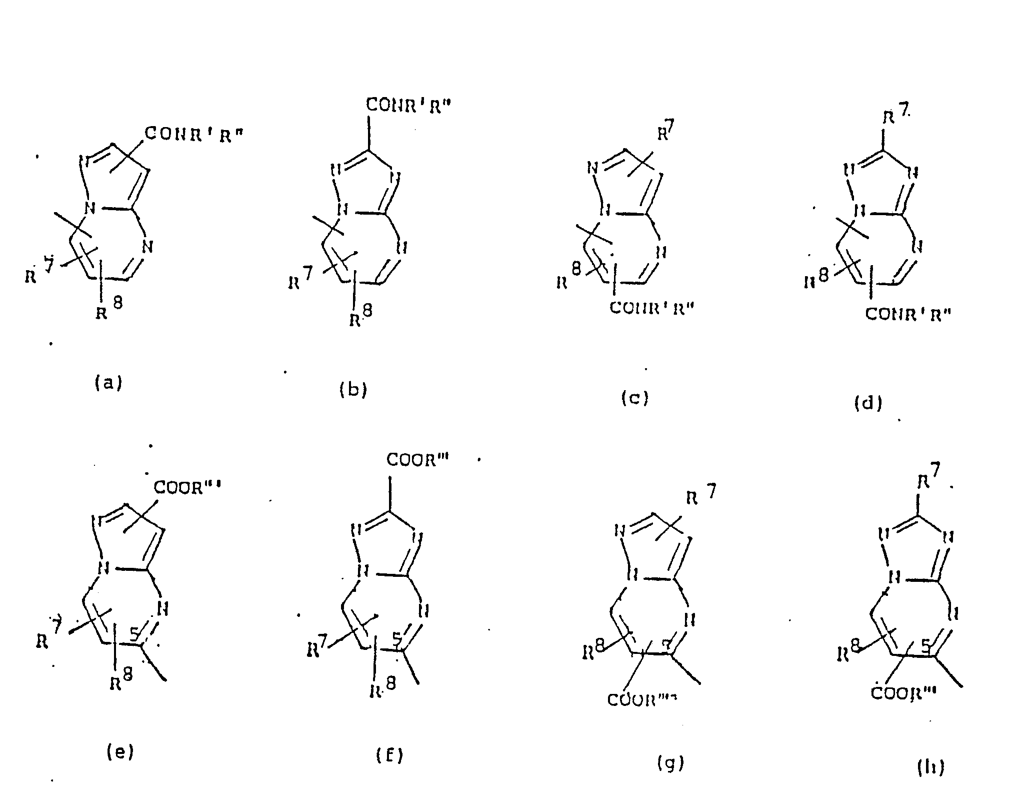 bedeutet, worin R⁷ und R⁸ je einzeln Wasserstoff, niederes Alkyl oder Trifluormethyl oder (in den Formeln (a), (b), (e) und (f)) zusammen Alkylen mit 3 oder 4 Kohlenstoffatomen darstellen, und Rʹ, Rʺ und R‴ je einzeln Wasserstoff, niederes Alkyl oder niederes Cycloalkyl bedeuten; wobei Verbindungen der Formel I, worin R⁴ und R⁵ zusammen eine zusätzliche Bindung darstellen, in der syn-isomeren Form vorliegen bzw. als Gemisch mit der anti-isomeren Form, worin die syn-isomere Form überwiegt, sowie von leicht hydrolysierbaren Estern und von Salzen dieser Verbindungen und von Hydraten der Verbindungen der Formel I bzw. von deren Estern und Salzen, dadurch gekennzeichnet, dass man
(a) eine Verbindung der allgemeinen Formel 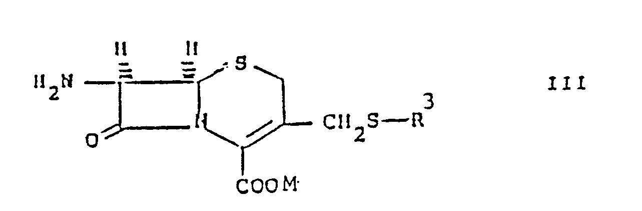 in der R³ die oben gegebene Bedeutung hat und M Wasserstoff oder eine Esterschutzgruppe darstellt,
mit einem Acylierungsmittel umsetzt und allfällige Schutzgruppe gewünschtenfalls abspaltet, oder dass man
(b) eine Verbindung der allgemeinen Formel 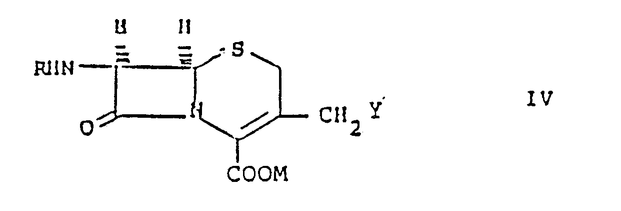 in der R und M die oben gegebene Bedeutung haben und Y eine Abgangsgruppe darstellt,
mit einer Verbindung der allgemeinen Formel
HSR³ V
in der R³ die oben gegebene Bedeutung hat, umsetzt und allfällige Schutzgruppen gewünschtenfalls abspaltet, oder dass man
(c) zur Herstellung von Verbindungen der Formel I, worin R die allgemeine Formel 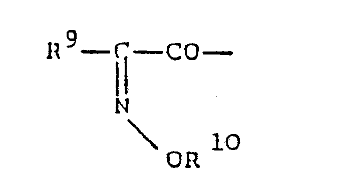 worin R⁹ und R¹⁰ die in Anspruch 2 gegebene Bedeutung haben,
hat, eine Verbindung der allgemeinen Formel 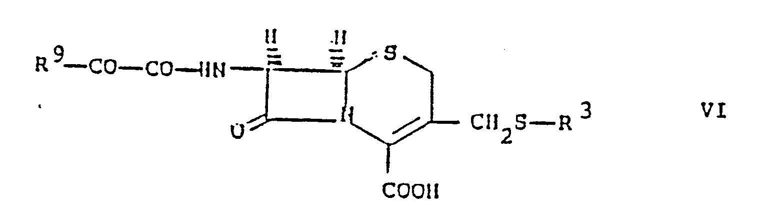 worin R³ die oben gegebene und R⁹ die in Anspruch 2 gegebene Bedeutung haben,
mit einem Salz einer Verbindung der allgemeinen Formel
NH₂OR¹⁰ VII
worin R¹⁰ die in Anspruch 2 gegebene Bedeutung hat, umsetzt, oder dass man
(d) zur Herstellung eines leicht hydrolysierbaren Esters einer Verbindung der Formel I eine Carbonsäure der Formel I einer entsprechenden Veresterung unterwirft, oder dass man
(e) zur Herstellung von Salzen und Hydraten eine Verbindung der Formel I bzw. Hydraten dieser Salze eine Verbindung der Formel I in ein Salz oder Hydrat bzw. ein Hydrat dieses Salzes überführt.

2. Verfahren zur Herstellung von Acylderivaten nach Anspruch 1 der allgemeinen Formel 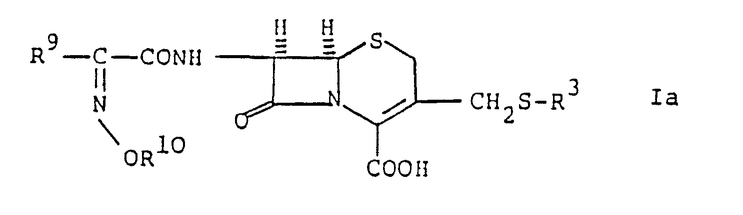 in der R³ die in Anspruch 1 angegebene Bedeutung hat, R⁹ einen gegebenenfalls substituierten 5- oder 6-gliedrigen Heterocyclus mit 1-4, vorzugsweise 1 oder 2 Heteroatomen, wie N, O, und/oder S, darstellt und R¹⁰ Wasserstoff, niederes Alkanoyl, niederes Alkyl , niederes Alkenyl, niederes Cycloalkyl, niederes Cycloalkenyl oder niederes Alkyl substituiert durch Carboxy, Carbamoyl, niederes Alkoxycarbonyl oder niederes Alkanoyl bedeutet, oder R¹⁰ einen Rest der allgemeinen Formeln
-P-Q
-P-CO-Q
-P-CO-NH-NH-CO-Q¹
bedeutet, worin P niederes Alkylen oder niederes Cycloalkylen bedeutet, Q eine der Gruppen 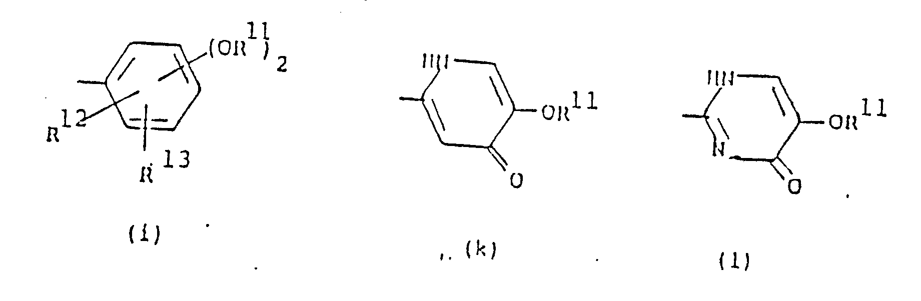 darstellt, worin R¹¹ Wasserstoff, niederes Alkanoyl oder tri(niederes Alkyl)-Silyl, R¹² Wasserstoff, -OR¹¹, niederes Alkoxy, Halogen, -OCOR¹⁴,-OCOOR¹⁴, -N(R¹⁴)₂, -NH-COR¹⁴, -NHCOOR¹⁴, -COR¹⁴, -SR¹⁴, -SOR¹⁴, -SO₂R¹⁴, -SO₃H, -COOR¹⁴, -CON(R¹⁴)₂ oder Nitro, R¹³ Wasserstoff oder Halogen und R¹⁴ Wasserstoff oder niederes Alkyl bedeutet, wobei in der Gruppe (i) die beiden Reste -OR¹¹ in 2,3- oder 3,4-Stellung stehen; und Q¹ eine der letztgenannten Gruppen (k) und (l) bedeutet, wobei die Verbindungen der Formel Ia in der syn-isomeren Form vorliegen bzw. als Gemisch mit der anti-isomeren Form, worin die syn-isomere Form überwiegt,
sowie von leicht hydrolysierbaren Estern und von Salzen dieser Verbindungen und von Hydraten der Verbindungen der Formel Ia bzw. von deren Estern und Salzen, dadurch gekennzeichnet, dass man entsprechend substituierte Ausgangsverbindungen einsetzt.

3. Verfahren zur Herstellung von Verbindungen nach Anspruch 1 oder 2, worin R³ eine der Gruppen 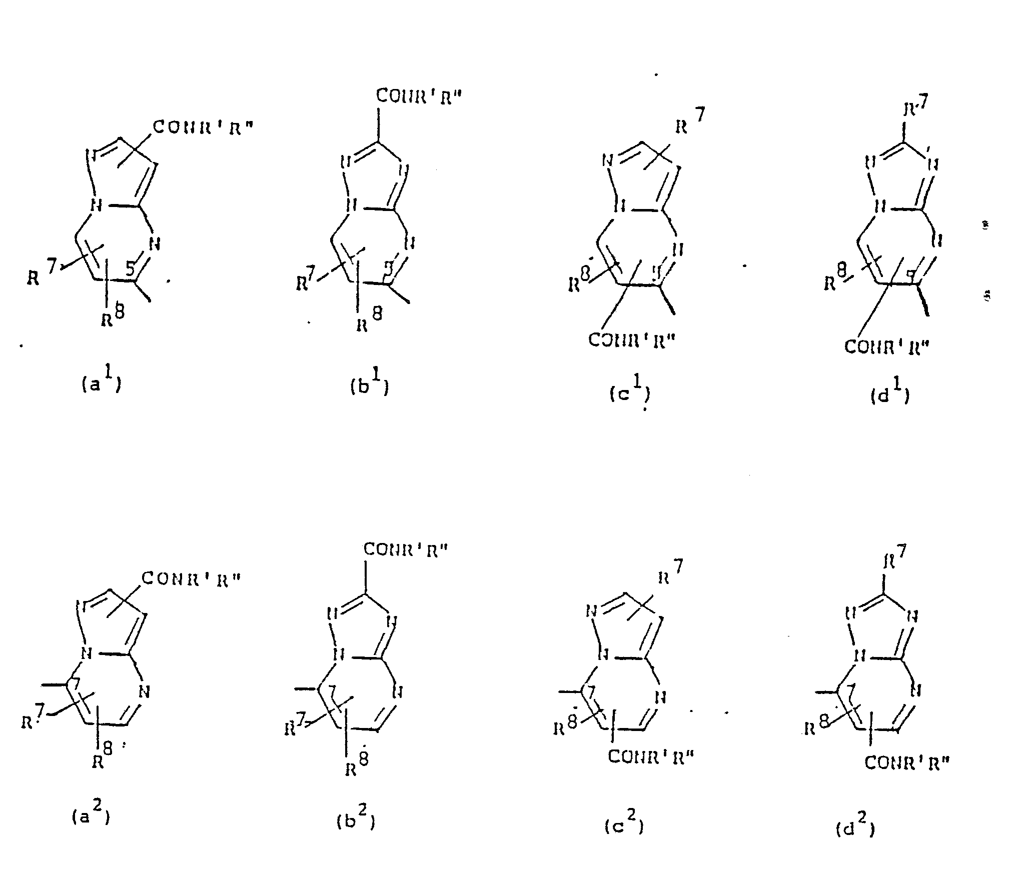 darstellt, worin Rʹ, Rʺ, R⁷ und R⁸ die in Anspruch 1 gegebene Bedeutung haben,
dadurch gekennzeichnet, dass man entsprechend substituierte Ausgangsverbindungen einsetzt.

4. Verfahren zur Herstellung von Verbindungen nach Anspruch 1 oder 2, worin R³ eine Gruppe 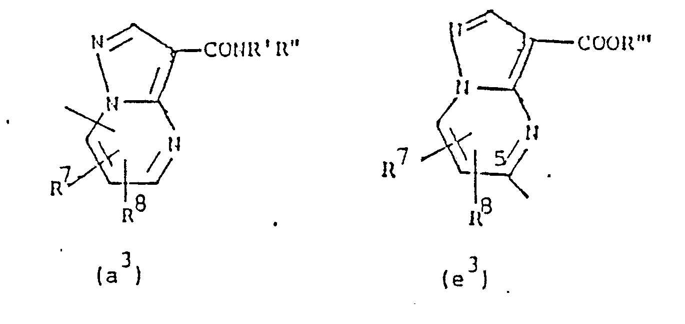 darstellt, worin Rʹ, Rʺ, R⁷ und R⁸ die in Anspruch 1 gegebene Bedeutung haben,
dadurch gekennzeichnet, dass man entsprechend substituierte Ausgangsverbindungen einsetzt.

5. Verfahren zur Herstellung von Verbindungen nach Anspruch 1 oder 2, worin R³ eine carbamoylsubstituierte bicyclische Gruppe der allgemeinen Formeln 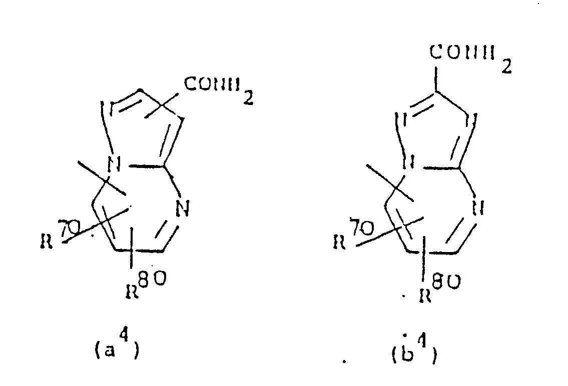 bedeutet, worin R⁷⁰ und R⁸⁰ je einzeln Wasserstoff, Methyl oder Trifluormethyl oder zusammen Alkylen mit 3 oder 4 Kohlenstoffatomen darstellen,
dadurch gekennzeichnet, dass man entsprechend substituierte Ausgangsverbindungen einsetzt.

6. Verfahren zur Herstellung von Verbindungen nach Anspruch 1 oder 2, worin R³ eine substituierte bicyclische Gruppe der allgemeinen Formeln 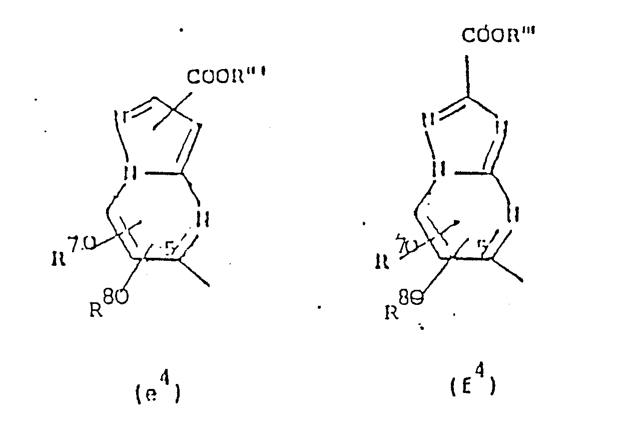 bedeutet, worin R⁷⁰ und R⁸⁰ je einzeln Wasserstoff, Methyl oder Trifluormethyl oder zusammen Alkylen mit 3 oder 4 Kohlenstoffatomen darstellen und R‴ Wasserstoff oder Methyl bedeutet,
dadurch gekennzeichnet, dass man entsprechend substituierte Ausgangsverbindungen einsetzt.

7. Verfahren zur Herstellung von Verbindungen nach Anspruch 1 oder 2, worin R³ eine der Gruppen 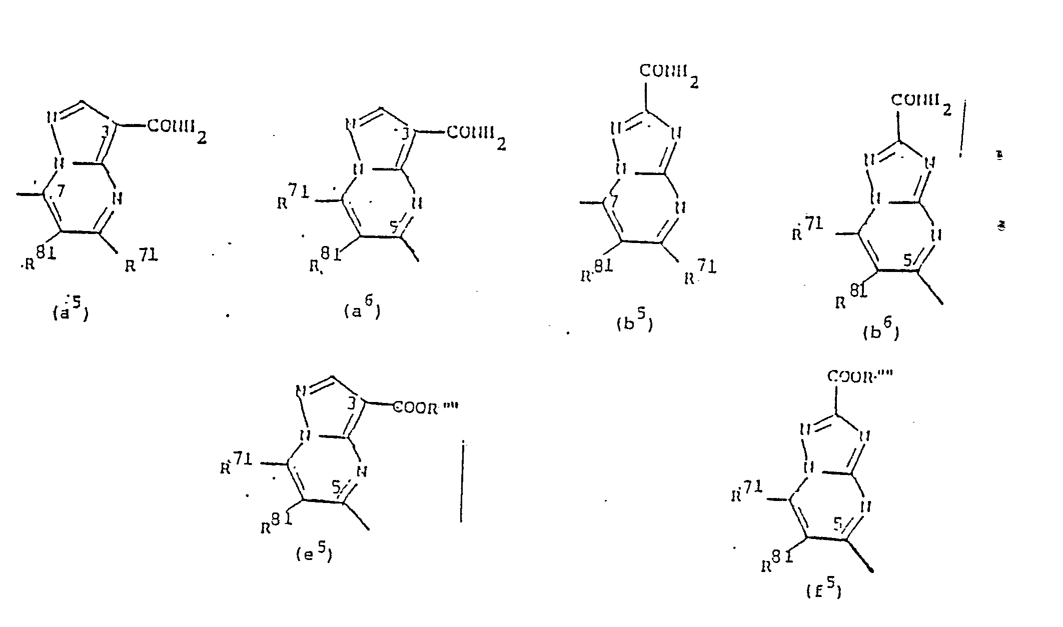 darstellt,
worin R⁷¹ Methyl oder Trifluormethyl und R⁸¹ Wasserstoff oder R⁷¹ und R⁸¹ zusammen Tetramethylen darstellen und Rʺʺ Wasserstoff oder Methyl bedeutet,
dadurch gekennzeichnet, dass man entsprechend substituierte Ausgangsverbindungen einsetzt.

8. Verfahren zur Herstellung von Verbindungen nach Anspruch 5, worin R³ die Gruppe (a⁴) darstellt, welche über die Stellung 5 verknüpft ist: 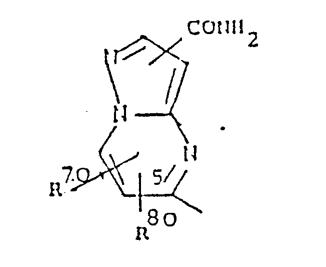 dadurch gekennzeichnet, dass man entsprechend substituierte Ausgangsverbindungen einsetzt.

9. Verfahren zur Herstellung von Verbindungen nach Anspruch 5, worin R³ die Gruppe (a⁴) darstellt, welche über die Stellung 7 verknüpft ist: 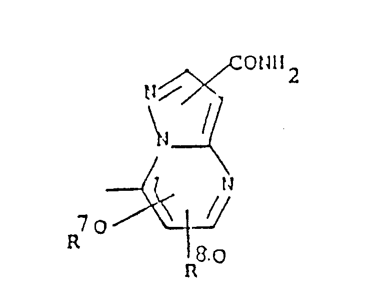 dadurch gekennzeichnet, dass man entsprechend substituierte Ausgangsverbindungen einsetzt.

10. Verfahren zur Herstellung von Verbindungen nach einem der Ansprüche 5, 8 und 9, worin R³ die Gruppe (a⁴) darstellt, in der die Carbamoylgruppe in Stellung 3 steht: 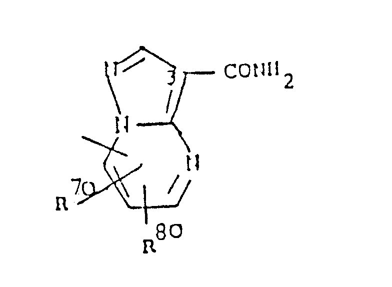 dadurch gekennzeichnet, dass man entsprechend substituierte Ausgangsverbindungen einsetzt.

11. Verfahren zur Herstellung von Verbindungen nach Anspruch 9 oder 10, worin R³ die Gruppe 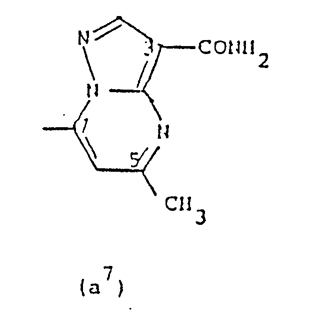 darstellt,
dadurch gekennzeichnet, dass man entsprechend substituierte Ausgangsverbindungen einsetzt.

12. Verfahren zur Herstellung von Verbindungen nach Anspruch 5, worin R³ die Gruppe (b⁴) darstellt, welche über die Stellung 5 verknüpft ist: 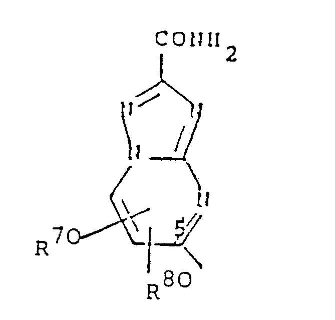 dadurch gekennzeichnet, dass man entsprechend substituierte Ausgangsverbindungen einsetzt.

13. Verfahren zur Herstellung von Verbindungen nach Anspruch 5, worin R³ die Gruppe (b⁴) darstellt, welche über die Stellung 7 verknüpft ist: 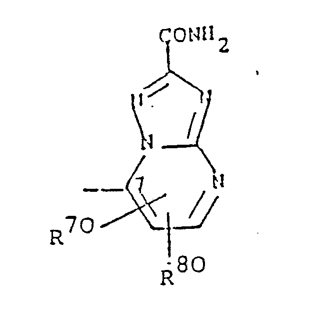 dadurch gekennzeichnet, dass man entsprechend substituierte Ausgangsverbindungen einsetzt.

14. Verfahren zur Herstellung von Verbindungen nach Anspruch 12 oder 13, worin R³ die Gruppe 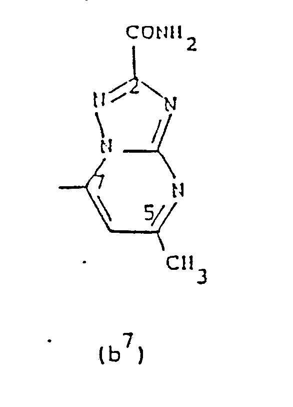 darstellt,
dadurch gekennzeichnet, dass man entsprechend substituierte Ausgangsverbindungen einsetzt.

15. Verfahren zur Herstellung von Verbindungen nach einem der Ansprüche 2-14, worin R⁹ 2-Amino-4-thiazolyl oder 5-Amino-3-thiadiazolyl darstellt, dadurch gekennzeichnet, dass man entsprechend substituierte Ausgangsverbindungen einsetzt.

16. Verfahren zur Herstellung von Verbindungen nach einem der Ansprüche 2-15, worin R¹⁰ Wasserstoff, niederes Alkanoyl, niederes Alkyl oder niederes Alkyl substituiert durch Carboxy, Carbamoyl, niederes Alkoxycarbonyl oder niederes Alkanoyl darstellt, dadurch gekennzeichnet, dass man entsprechend substituierte Ausgangsverbindungen einsetzt.

17. Verfahren zur Herstellung von Verbindungen nach Anspruch 16, worin R¹⁰ Methyl Allyl, Carboxymethyl, Carbamoylmethyl, 1-Carboxy-1-methyläthyl oder Pivaloyl darstellt, dadurch gekennzeichnet, dass man entsprechend substituierte Ausgangsverbindungen einsetzt.

18. Verfahren zur Herstellung von Verbindungen nach einem der Ansprüche 2-15, worin R¹⁰ eine der Gruppen
-P¹-Q
-P¹-CO-Q
-P¹-CO-NH-NH-CO-Q¹
worin Q und Q¹ die in Anspruch 2 gegebene Bedeutung haben und P¹ die Gruppe -CH₂- oder 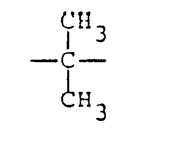 bedeutet,
darstellt,
dadurch gekennzeichnet, dass man entsprechend substituierte Ausgangsverbindungen einsetzt.

19. Verfahren zur Herstellung von Verbindungen nach Anspruch 18, worin R¹⁰ eine der Gruppen 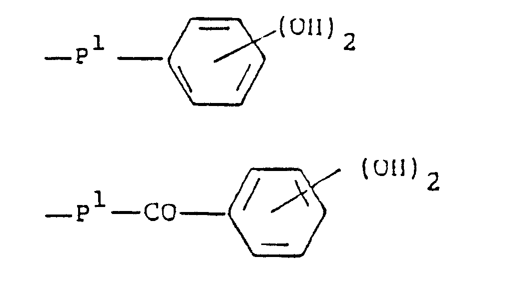 worin P¹ die in Anspruch 18 gegebene Bedeutung hat und die beiden Hydroxygruppen in 2,3- oder 3,4-Stellung stehen,
darstellt, dadurch gekennzeichnet, dass man entsprechend substituierte Ausgangsverbindungen einsetzt.

20. Verfahren zur Herstellung von Verbindungen nach Anspruch 18, worin R¹⁰ eine der Gruppen 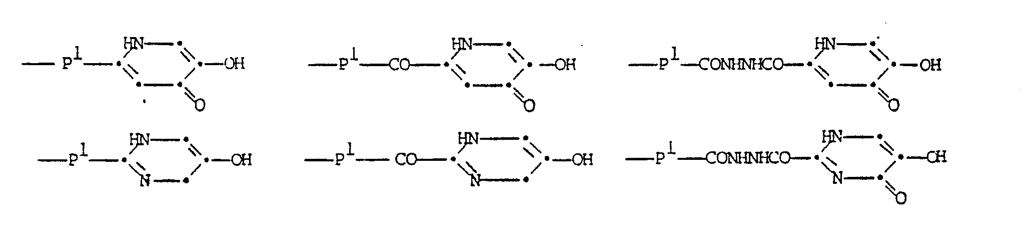 worin P¹ die in Anspruch 18 gegebene Bedeutung hat, darstellt, dadurch gekennzeichnet, dass man entsprechend substituierte Ausgangsverbindungen einsetzt.

21. Verfahren nach Anspruch 5 zur Herstellung von
(6R,7R)-7-[(Z)-2-(2-Amino-4-thiazolyl)-2-(methoxyimino]-acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[(t-butoxycarbonyl)methoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[(pivaloyloxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]-oct-2-en-2-carbonsäure,
(6R,7R)-7-[(Z)-2-(2-Amino-4-thiazolyl)-2-[[1-(t-butoxycarbonyl)-1-methyläthoxy]imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[(carboxymethoxy)imino]acetamido]-3-[(2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[1-(carboxy-1-methyläthoxy)imino]acetamido]-3-[[[2-carbamoyl-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[(carbamoylmethoxy)imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure sowie von Salzen dieser Verbindungen,
dadurch gekennzeichnet, dass man entsprechend substituierte Ausgangsverbindungen einsetzt.

22. Verfahren nach Anspruch 5 zur Herstellung von (6R,7R)-7-[(Z)-2-(2-Amino-4-thiazolyl)-2-[[1-[3-[(1,4-dihydro-5-hydroxy-4-oxo-2-pyridyl)carbonyl]carbazoyl]-1-methyläthoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[(1,4-dihydro-5-hydroxy-4-oxo-2-pyrimidinyl)methoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure
sowie von Salzen dieser Verbindungen, dadurch gekennzeichnet, dass man entsprechend substituierte Ausgangsverbindungen einsetzt.

23. Verfahren zur Herstellung von pharmazeutischen Präparaten, dadurch gekennzeichnet, dass man ein in Anspruch 1 definiertes Acylderivat der Formel I oder ein leicht hydrolysierbarer Ester oder Salz einer dieser Acylderivate oder ein Hydrat einer Verbinding der Formel I bzw. eines Esters oder Salzes davon als wirksamen Bestandteil mit zur therapeutischen Verabreichung geeigneten, nicht-toxischen, inerten, an sich in solchen Präparaten üblichen festen und flüssigen Trägern und/oder Excipientien vermischt.

24. Verwendung von Verbindungen gemäss einem der Ansprüche 1-22 bei der Behandlung bzw. Prophylaxe von Krankheiten.

25. Verwendung von Verbindungen gemäss einem der Ansprüche 1-22 bei der Behandlung bzw. Prophylaxe von Infektionskrankheiten.

26. Verwendung von Verbindungen gemäss einem der Ansprüche 1-22 bei der Herstellung von Arzneimitteln für die Behandlung bzw. Prophylaxe von Infektionskrankheiten.

27. Verbindungen der allgemeinen Formel 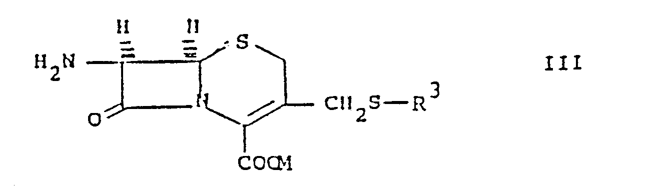 in der R³ die in Anspruch 1 gegebene Bedeutung hat und M Wasserstoff oder eine Esterschutzgruppe darstellt.

28. Verbindungen der allgemeinen Formel
HSR³ V
in der R³ die in Anspruch 1 gegebene Bedeutung hat.

29. Verbindungen der allgemeinen Formel 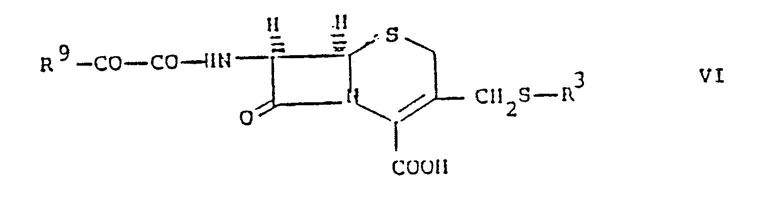 worin R³ die in Anspruch 1 gegebene Bedeutung und R⁹ die in Anspruch 2 gegebene Bedeutung hat.
